# EUROPÄISCHE PATENTANMELDUNG

(11) **EP 4 506 000 A1**
(43) Veröffentlichungstag der Anmeldung: **12.02.2025**
(21) Anmeldenummer: 23190191.9
(22) Anmeldetag: 08.08.2023
(51) Int. Cl.: A61K 31/375, A61K 33/20, A61P 17/00, A61P 17/02, A61P 31/02

(54) **ANTISEPTISCHES KIT UND DESSEN VERWENDUNG**

(71) Anmelder: Heraeus Medical GmbH, 61273 Wehrheim (DE)
(72) Erfinder: Vogt, Sebastian, Dr., 61273 Wehrheim (DE); Kluge, Thomas, Dr., 61273 Wehrheim (DE)
(74) Vertreter: Heraeus IP

(57) **Zusammenfassung**

Die vorliegende Erfindung betrifft ein Kit zur Verwendung bei der lokalen antiseptischen Behandlung eines Patienten, wobei das Kit ein antiseptisches Oxidationsmittel und ein Reduktionsmittel umfasst, wobei das antiseptische Oxidationsmittel durch Reaktion mit dem Reduktionsmittel zersetzbar ist.

## Beschreibung

### GEBIET DER ERFINDUNG

Die vorliegende Erfindung betrifft das Gebiet der Medizintechnik, insbesondere Vorrichtungen zur Präparation und Abgabe von Wirkstofflösungen, beispielsweise Wundspüllösungen. Die vorliegende Erfindung betrifft weiterhin die medizinische Verwendung von wässrigen Lösungen zur lokalen antiseptischen Behandlung und antiseptische Kits. Die erfindungsgemäßen wässrigen Lösungen, Vorrichtungen und Kits sind zur antiseptischen Behandlung von Weichgewebe- und Knochengewebeoberflächen verwendbar.

Antiseptische Hypochlorit-Lösungen sind im Stand der Technik bekannt. Seit über einhundert Jahren wird eine 4-5 %ige Natriumhypochlorit-Lösung zur Desinfektion von Hautoberflächen und zur Desinfektion von Gegenständen verwendet. Dieses Desinfektionsmittel ist als Dakin'sche Lösung allgemein bekannt. Ebenso ist die Verwendung von Calciumhypochlorit und von Chlorkalk allgemeiner Stand der Technik. Bei Hypochlorit-Lösungen wirken die Hypochlorit-Ionen selbst und besonders bei pH-Werten kleiner pH 7 die im Gleichgewicht zu den Hypochlorit-Ionen stehende hypochlorige Säure antiseptisch. Hypochlorit-Ionen und auch die hypochlorige Säure wirken als Oxidationsmittel durch Übertragung von Sauerstoff auf die zu behandelnden Mikroorganismen oder auch Biofilme ein.

Eine Vielzahl von Desinfektionsmitteln auf Grundlage von Hypochlorit wurden beschrieben. Exemplarisch sind dafür folgende Patentdokumente: US8945630B2, US2011/0236490A1, WO2021001789, US2019328776A1, WO2021134041A1, WO2022/038507A1, WO2020252433, WO2020174436A1, US243781, US3749672, US6162371, WO1991/003936, US6471974, US2009/0258083, WO2010148004, WO2010148004, US2009/0148342A1, WO2016/100543A2, WO2020/089693A1, und WO2021/162736A1.

Bei der Verwendung von Hypochlorit-Lösungen besteht die Möglichkeit, dass nicht die gesamte Menge an Hypochlorit während der eigentlichen Desinfektion verbraucht wird und Reste des Hypochlorits auf den zu desinfizierenden Oberflächen, vor allem auf Oberflächen humaner oder tierischer Gewebe, verbleiben. Dadurch können lokal unerwünschte Wirkungen, insbesondere bei höher konzentrierten Hypochlorit-Lösungen, nicht ausgeschlossen werden.

Daher kann es vorteilhaft sein, die Verwendung von antiseptischen Lösungen von Sauerstoff-abspaltenden Antiseptika so zu verbessern, dass nach Einwirkung der antiseptischen oxidierenden Lösungen auf Oberflächen von humanem oder tierischen Gewebe keine Reste der aktiven Antiseptika auf den Oberflächen verbleiben, um unerwünschte Wirkungen der Oxidationsmittelreste sicher zu vermeiden.

### BEVORZUGTE AUSFÜHRUNGSFORMEN

Eine Aufgabe der vorliegenden Erfindung besteht darin, eines oder mehrere der oben geschilderten und weitere Probleme des Stands der Technik zu lösen. Beispielsweise liefert die Erfindung in einer Ausführungsform eine wirkstoffhaltige Vorrichtung, mit dessen Hilfe sich eine gebrauchsfertige Wirkstofflösung mithilfe handelsüblicher Behälter mit steriler Kochsalzlösung oder vergleichbaren Lösungen schnell, einfach und kostengünstig herstellen lässt. Wenn die erfindungsgemäße Vorrichtung den Wirkstoff in fester Form enthält, kann die Vorrichtung längere Zeit gelagert werden, auch wenn sie einen Wirkstoff enthält, der in wässriger Lösung nicht dauerhaft stabil ist. Zudem entfällt bei der Herstellung einer solchen Vorrichtung die Sterilisation von Flüssigkeiten. Einige Wirkstoffe lassen sich in fester Form besser sterilisieren, da sie sich in Lösung während der Sterilisierung leichter zersetzen. In einigen Ausführungsformen liefert die erfindungsgemäße Vorrichtung die Möglichkeit, die darin hergestellte Flüssigkeit direkt an einen Patienten abzugeben, beispielsweise mithilfe einer Düse, die an der erfindungsgemäßen Vorrichtung angebracht werden kann. In einigen Ausführungsformen liefert die Erfindung ein Behandlungsverfahren und ein Kit zur Anwendung in einem solchen Verfahren, bei dem der antiseptische Wirkstoff nach der Anwendung am Patienten durch ein Reduktionsmittel neutralisiert wird. Die antiseptischen Lösungen sind bevorzugt so zusammengesetzt, dass diese sich zu ungiftigen Zerfallsprodukten umsetzen können. Weiterhin sind die Lösungen bevorzugt so zusammengesetzt, dass diese nicht hypotonisch sind und ein Platzen von humanen oder tierischen Zellen während der desinfizierenden Behandlung durch eine Aufnahme von Wasser vermieden wird. Die Lösungen weisen bevorzugt keine pH-Werte kleiner pH 4 und keine pH-Werte größer 8 auf, um Schädigungen des zu desinfizierenden Gewebes so weit als möglich zu vermeiden. Demnach können die Lösungen bevorzugt einen pH-Wert von 4 bis 8, zum Beispiel von 6 bis 7,5 oder von 7,3 bis 7,6, aufweisen.

Diese Aufgaben werden gelöst durch die hierin beschriebenen Verfahren, Vorrichtungen, Kits und medizinischen Verwendungen, insbesondere denjenigen, die in den Patentansprüchen beschrieben sind. Bevorzugte Ausführungsformen der Erfindung werden nachfolgend beschrieben.

Eine erste Ausführungsform betrifft eine Vorrichtung zur Herstellung einer Wirkstofflösung, bevorzugt einer antiseptischen Wundspüllösung, aufweisend
- ein Anschlusselement, das zum flüssigkeitsdichten Anschluss an einen Behälter ausgebildet und eingerichtet ist;
- einen Dorn, der zum Durchstechen eines Septums eines Behälters ausgebildet und eingerichtet ist,
- ein Reservoir, das einen Wirkstoff in fester Form enthält, und über einen Ausgang des Reservoirs fluidleitend mit dem Anschlusselement verbindbar ist;
- einen flüssigkeitsdurchlässigen Filter, der an dem Ausgang des Reservoirs angeordnet ist, und eingerichtet ist, den Wirkstoff in fester Form in dem Reservoir zurückzuhalten;
- ein Fluidikelement, welches zur Aufnahme einer Flüssigkeit aus einem Behälter in das Reservoir eingerichtet ist, und/oder zur Abgabe einer Flüssigkeit aus dem Reservoir eingerichtet ist;
wobei die Vorrichtung ausgebildet und eingerichtet ist, eine Flüssigkeit aus einem Behälter in das Reservoir aufzunehmen, den Wirkstoff in der Flüssigkeit zu lösen, und den gelösten Wirkstoff über das Anschlusselement an einen Patienten abzugeben.

Eine zweite Ausführungsform betrifft eine Vorrichtung nach Ausführungsform 1, wobei der Dorn einen hohlen Innenraum aufweist, der zur Aufnahme einer Flüssigkeit aus einem Behälter in das Reservoir eingerichtet ist.

Eine dritte Ausführungsform betrifft eine Vorrichtung nach einer der vorangehenden Ausführungsformen, wobei das Fluidikelement einen innerhalb des Reservoirs beweglich angeordneten Kolben aufweist, der das Reservoir flüssigkeitsdicht abschließt.

Eine vierte Ausführungsform betrifft eine Vorrichtung nach einer der vorangehenden Ausführungsformen, welche ein erstes Bauteil und ein zweites Bauteil aufweist, welche lösbar miteinander verbindbar sind.

Eine fünfte Ausführungsform betrifft eine Vorrichtung nach Ausführungsform 4, wobei das erste Bauteil das Anschlusselement und den Dorn enthält, und/oder wobei das zweite Bauteil das Reservoir, den Filter und das Fluidikelement enthält.

Eine sechste Ausführungsform betrifft eine Vorrichtung nach Ausführungsform 4 oder 5, wobei die Vorrichtung weiterhin ein Gewinde aufweist, mit dessen Hilfe das erste Bauteil mit dem zweiten Bauteil verbindbar ist.

Eine siebte Ausführungsform betrifft eine Vorrichtung nach einer der vorangehenden Ausführungsformen, wobei die Vorrichtung vollständig sterilisiert ist, bevorzugt vollständig pyrogenfrei ist.

Eine achte Ausführungsform betrifft eine Vorrichtung nach einer der vorangehenden Ausführungsformen, wobei die Vorrichtung weiterhin eine Spritzdüse aufweist, welche zur Abgabe des Wirkstoffs in gelöster Form eingerichtet ist.

Eine neunte Ausführungsform betrifft eine Vorrichtung gemäß Ausführungsform 8, wobei die Spritzdüse lösbar fluidleitend mit dem Anschlusselement verbindbar ist.

Eine zehnte Ausführungsform betrifft eine Vorrichtung nach einer der vorangehenden Ausführungsformen, wobei der Wirkstoff ein Antiseptikum ist, welches bevorzugt ausgewählt ist aus der Gruppe bestehend aus Calciumhypochlorit, Magnesiumhypochlorit, Natriumhypochlorit, Natriumpercarbonat, Natriumperoxid, Calciumperoxid, Harnstoff-Peroxid, Triisocyanurchlorid, Natrium-N-chlor-4-methylbenzolsulfonamid, Natrium-N-chlorbenzolsulfonamid, Octenidin, Polyhexanid und Chlorhexidindigluconat.

Eine elfte Ausführungsform betrifft eine Vorrichtung nach einer der vorangehenden Ausführungsformen, wobei das Anschlusselement eine erste Durchführung aufweist, welche zur Aufnahme einer Flüssigkeit aus einem Behälter in das Reservoir eingerichtet ist.

Eine zwölfte Ausführungsform betrifft eine Vorrichtung nach einer der vorangehenden Ausführungsformen, wobei das Anschlusselement eine zweite Durchführung aufweist, welche zur Abgabe einer Flüssigkeit aus einem Behälter eingerichtet ist, wobei die Abgabe bevorzugt in einem Zustand möglich ist, in welchem die Vorrichtung über die erste Durchführung gemäß Ausführungsform 11 mit dem Reservoir verbunden ist.

Eine dreizehnte Ausführungsform betrifft eine Vorrichtung nach einer der vorangehenden Ausführungsformen, wobei das Reservoir durchlässig für Gammastrahlen und/oder Elektronenstrahlen ist.

Ein weiterer Aspekt betrifft ein Kit, aufweisend eine Vorrichtung nach einem der Ausführungsformen 1 bis 13 und einen Behälter mit einer medizinisch verträglichen Flüssigkeit, wobei die Vorrichtung lösbar mit dem Behälter verbindbar ist, wobei das Kit gemeinsam keimdicht verpackt, bevorzugt vollständig sterilisiert, weiter bevorzugt vollständig pyrogenfrei ist.

Ein weiterer Aspekt betrifft einen Wirkstoff zur Verwendung in einem medizinischen Behandlungsverfahren, wobei der Wirkstoff mithilfe einer Vorrichtung nach einem der vorangegangenen Ausführungsformen unmittelbar vor der Verabreichung an einen Patienten in einer Flüssigkeit gelöst und anschließend in gelöster Form verabreicht wird.

Ein weiterer Aspekt betrifft ein Verfahren zur Herstellung einer Wirkstofflösung, welches die nachfolgenden Schritte umfasst,
a) Aufnahme einer Flüssigkeit aus einem Behälter mithilfe einer Vorrichtung nach einer der Ausführungsformen 1 bis 13 in das Reservoir der Vorrichtung,
b) Mischen des Wirkstoffs innerhalb des Reservoirs mit der in Schritt a) aufgenommenen Flüssigkeit,
c) dadurch Erhalt einer Wirkstofflösung,
d) gegebenenfalls Abgabe der Wirkstofflösung in den Behälter.

Ein weiterer Aspekt betrifft die Verwendung einer Vorrichtung nach einer der Ausführungsformen 1 bis 13 zur Herstellung einer Wirkstofflösung.

Ein weiterer Aspekt betrifft ein Kit zur Verwendung bei der lokalen antiseptischen Behandlung eines Patienten, wobei das Kit ein antiseptisches Oxidationsmittel und ein Reduktionsmittel umfasst, wobei das antiseptische Oxidationsmittel durch Reaktion mit dem Reduktionsmittel zersetzbar ist.

In einer zweiten Ausführungsform des Kits zur Verwendung gemäß dieses Aspekts umfasst die Verwendung ein Inkontaktbringen des Oxidationsmittels mit einer Gewebeoberfläche des Patienten und ein nachfolgendes Inkontaktbringen des Reduktionsmittels mit der Gewebeoberfläche des Patienten.

In einer dritten Ausführungsform des Kits zur Verwendung gemäß dieses Aspekts erfolgt das Inkontaktbringen des Oxidationsmittels und/oder des Reduktionsmittels mit der Gewebeoberfläche des Patienten jeweils für eine Zeitdauer von 10 Sekunden bis 300 Sekunden.

In einer vierten Ausführungsform des Kits zur Verwendung gemäß dieses Aspekts umfasst das Kit eine Stoffmenge des Reduktionsmittels, welche mindestens der Stoffmenge des Oxidationsmittels entspricht.

In einer fünften Ausführungsform des Kits zur Verwendung gemäß dieses Aspekts ist das Oxidationsmittel ausgewählt aus der Gruppe bestehend aus Natriumhypochlorit (CAS 7681-52-9), Calciumhypochlorit (CAS 7778-54-3) und Natriumchlorit (CAS 7758-19-2).

In einer sechsten Ausführungsform des Kits zur Verwendung gemäß dieses Aspekts ist das Reduktionsmittel ausgewählt aus der Gruppe bestehend aus Ascorbinsäure (CAS 50-81-7), Natriumascorbat (CAS 134-03-2), Kaliumascorbat (CAS 15421-15-5), Calciumascorbat (CAS 5743-27-1), L-Cystin (CAS 52-90-4) und Cysteamin (CAS 60-23-1).

In einer siebten Ausführungsform des Kits zur Verwendung gemäß dieses Aspekts umfasst das Kit weiterhin eine Puffersubstanz, wobei die Puffersubstanz bevorzugt ausgewählt ist aus der Gruppe bestehend aus Citronensäure (CAS 77-92-9), Natriumdihydrogencitrat (CAS 18996-35-5), Dinatriumhydrogencitrat (CAS 6132-05-4), Natriumdihydrogenphosphat (CAS 7558-80-7), Calciumdihydrogenphosphat (CAS 7758-23-8), Natriumhydrogensulfat (CAS 7681-38-1), Dinatriumhydrogenphosphat (CAS 7558-79-4) und Trinatriumphosphat (CAS 760-54-9), besonders bevorzugt Citronensäure.

In einer achten Ausführungsform des Kits zur Verwendung gemäß dieses Aspekts umfasst das Kit eine erste Lösung mit Hypochlorit-Ionen, Chloritionen und/oder hypochloriger Säure in einer Konzentration von insgesamt (kumulativ) 50 ppm bis 5000 ppm.

In einer neunten Ausführungsform des Kits zur Verwendung gemäß dieses Aspekts umfasst das Kit eine zweite Lösung mit Ascorbinsäure und/oder Ascorbat-Ionen in einer Konzentration von 50 ppm bis 10.000 ppm.

In einer zehnten Ausführungsform des Kits zur Verwendung gemäß dieses Aspekts umfasst das Kit eine erste Lösung mit dem Oxidationsmittel und/oder eine zweite Lösung mit einem Reduktionsmittel mit einer Osmolarität von jeweils mindestens 310 mOsmol/L.

In einer elften Ausführungsform des Kits zur Verwendung gemäß dieses Aspekts umfasst das Kit eine erste Lösung mit einem Oxidationsmittel und einem pH-Wert im Bereich von pH 4 bis pH 8.

In einer zwölften Ausführungsform des Kits zur Verwendung gemäß dieses Aspekts umfasst das Kit eine zweite Lösung mit einem Reduktionsmittel und einem pH-Wert im Bereich von pH 4 bis pH 7.

In einer dreizehnten Ausführungsform des Kits zur Verwendung gemäß dieses Aspekts wird/werden eine erste Lösung mit dem Oxidationsmittel und/oder eine zweite Lösung mit dem Reduktionsmittel jeweils unmittelbar vor dem Inkontaktbringen mit der Gewebeoberfläche durch Lösen des Oxidationsmittels bzw. Reduktionsmittels in fester Form, gegebenenfalls gemeinsam mit einer Puffersubstanz, in Wasser hergestellt.

In einer vierzehnten Ausführungsform des Kits zur Verwendung gemäß dieses Aspekts umfasst die Verwendung die Behandlung eines Krankheitszustands, der ausgewählt ist aus der Gruppe bestehend aus
a) Freiliegende Oberflächen von künstlichen Gelenken und gelenknahen Knochen- und Weichgeweben, bevorzugt im Rahmen von DAIR-Prozeduren,
b) Wunden mit freiliegendem Knochengewebe, Knorpelgewebe, Bändern und Sehnen,
c) Intra- und postoperative Operationswunden,
d) chronischen Wunden,
e) Dekubiti,
f) Diabetische Ulcera,
g) Ulcus cruris,
h) Bisswunden,
i) Schnittwunden,
j) Stichwunden,
k) Schnittwunden,
l) Risswunden,
m) Schürfwunden,
n) Schussverletzungen,
o) Splitterverletzungen,
p) Fisteln,
q) Abszesse und
r) Verbrennungen ersten und zweiten Grades.

In einer fünfzehnten Ausführungsform des Kits zur Verwendung gemäß dieses Aspekts umfasst das Kit weiterhin ein Tensid, wobei die Verwendung das Inkontaktbringen des Tensids gemeinsam mit dem Oxidationsmittel mit einer Gewebeoberfläche des Patienten umfasst, wobei das Tensid bevorzugt ein Alkylsulfat ist.

### KURZE BESCHREIBUNG DER ZEICHNUNGEN

**Figur 1** zeigt einen ersten Teil einer Vorrichtung, welche mit einem Behälter verbindbar ist.
**Figur 2** zeigt einen Behälter, der mit einer erfindungsgemäßen Vorrichtung verbunden werden kann.
**Figur 3** zeigt einen ersten Teil einer erfindungsgemäßen Vorrichtung, welche an einen Behälter angebracht ist.
**Figur 4** zeigt einen zweiten Teil einer erfindungsgemäßen Vorrichtung mit einem Reservoir für einen Wirkstoff und einem Fluidikelement zum Transport einer Flüssigkeit aus einem Behälter.
**Figur 5** zeigt eine erfindungsgemäße Vorrichtung, die mit einem Behälter verbunden ist.
**Figur 6** zeigt einen ersten Teil einer erfindungsgemäßen Vorrichtung, welcher ein Gewinde zur Verbindung mit einem Verschluss oder einem zweiten Teil der Vorrichtung aufweist.
**Figur 7** zeigt einen ersten Teil einer erfindungsgemäßen Vorrichtung gemäß Figur 6, der über das Gewinde mit einem zweiten Teil der Vorrichtung verbunden ist.
**Figur 8** zeigt einen ersten Teil einer erfindungsgemäßen Vorrichtung gemäß Figur 6, der über das Gewinde mit einer Düse verbunden ist.
**Figur 9** zeigt eine Vorrichtung, die mit einer Abgabevorrichtung verbunden ist.
**Figur 10** zeigt eine Vorrichtung, die mit einer Düse und einem Schild versehen ist.
**Figur 11** zeigt eine Düse mit einem Gelenk, die mit einer erfindungsgemäßen Vorrichtung verwendbar ist.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist. Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse, Vorrichtungen, Kits und Verwendungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Eine erste Ausführungsform beschreibt eine Vorrichtung zur Herstellung einer Wirkstofflösung, aufweisend
- ein Anschlusselement, das zum flüssigkeitsdichten Anschluss an einen Behälter ausgebildet und eingerichtet ist;
- einen Dorn, der zum Durchstechen eines Septums eines Behälters ausgebildet und eingerichtet ist;
- ein Reservoir, das einen Wirkstoff in fester Form enthält, und über einen Ausgang des Reservoirs fluidleitend mit dem Anschlusselement verbindbar ist;
- einen flüssigkeitsdurchlässigen Filter, der an dem Ausgang des Reservoirs angeordnet ist, und eingerichtet ist, den Wirkstoff in fester Form in dem Reservoir zurückzuhalten;
- ein Fluidikelement, welches zur Aufnahme einer Flüssigkeit aus einem Behälter in das Reservoir eingerichtet ist, und/oder zur Abgabe einer Flüssigkeit aus dem Reservoir eingerichtet ist;
wobei die Vorrichtung ausgebildet und eingerichtet ist, eine Flüssigkeit aus einem Behälter in das Reservoir aufzunehmen, den Wirkstoff in der Flüssigkeit zu lösen, und den gelösten Wirkstoff über das Anschlusselement an einen Patienten abzugeben.

Die Vorrichtung umfasst einen Wirkstoff. Der Wirkstoff ist bevorzugt ein antiseptischer Wirkstoff, zum Beispiel ein antiseptisches Oxidationsmittel, wie nachfolgend ausführlicher beschrieben. Die Wirkstofflösung ist bevorzugt eine antiseptische Wundspüllösung. Die Begriffe "antiseptisch" und "antimikrobiell" werden hierin synonym verwendet, und bezeichnen eine bakterizide oder fungizide, d. h. keimtötende, Wirkung eines Wirkstoffs. Die Vorrichtung kann weiterhin einen Hilfsstoff enthalten. Der Wirkstoff kann als Gemisch mit einem solchen Hilfsstoff vorliegen. Der Hilfsstoff kann beispielsweise eine Puffersubstanz umfassen. In einer Ausführungsform ist der Hilfsstoff ausgewählt aus der Gruppe bestehend aus Glucuronsäure, Galacturonsäure, Gluconsäurelacton, Natriumgluconat, Natriumhydrogensulfat, Natriumdihydrogenphosphat, Calciumdihydrogenphosphat, Citronensäure und Mononatriumcitrat.

Die Vorrichtung weist ein Anschlusselement auf. Das Anschlusselement ist zum flüssigkeitsdichten Anschluss der Vorrichtung an einen Behälter ausgebildet und eingerichtet. Solche Behälter können beispielsweise Verpackungen sein, in denen physiologische Kochsalzlösungen, Infusionslösungen und ähnliche flüssige Medizinprodukte handelsüblich erhältlich sind. Gebräuchlich hierfür sind beispielsweise Flaschen oder Folienbeutel. Diese Behälter bestehen häufig aus Kunststoff. Der Behälter kann ein Behälter mit flexibler, bevorzugt elastisch verformbarer, Außenwand sein, sodass eine darin enthaltene Flüssigkeit durch Zusammendrücken des Behälters aus dem Behälter freigesetzt werden kann. Der Behälter kann ein Septum umfassen, welches mit dem Dorn der Vorrichtung durchstochen werden kann. Das Anschlusselement der Vorrichtung kann bevorzugt formschlüssig mit einem Behälter verbindbar sein. Beispielsweise kann das Anschlusselement an die Öffnung einer Kunststoffflasche oder den Anschluss eines Folienbeutels aufsteckbar sein. Die Vorrichtung enthält weiterhin einen Dorn, der zum Durchstechen eines Septums eines Behälters ausgebildet und eingerichtet ist. Der Dorn kann hierzu eine spitz zulaufende Form aufweisen, mit dessen Spitze ein Septum eines Behälters durch manuelles Ausüben von Druck des Dorns auf das Septum durchdrungen werden kann. Vergleichbare Strukturen werden zum Anschluss von Infusionbestecken an Behälter mit Infusionslösungen verwendet.

Die Vorrichtung weist weiterhin ein Reservoir auf. Das Reservoir enthält einen Wirkstoff in fester Form, beispielsweise als Pulver. Das Reservoir kann eine im wesentlichen zylindrische Form aufweisen, ähnlich dem Innenraum einer Spritze.

Die Vorrichtung kann eingerichtet sein, den enthaltenen Wirkstoff in gelöster Form in den Behälter abzugeben.

An einem Ausgang des Reservoirs ist ein Filter angeordnet. Dieser Filter ist eingerichtet, den Wirkstoff in dem Reservoir zurückzuhalten, solange der Wirkstoff nicht in einer Flüssigkeit gelöst ist. Auf diese Weise kann verhindert werden, dass Wirkstoff in ungelöster Form an einen Patienten abgegeben wird. Erwünscht ist hingegen, dass der Wirkstoff erst in ausschließlich gelöster Form an einen Patienten abgegeben werden kann. Hierzu kann der Filter bevorzugt flüssigkeitsdurchlässig sein. Der Filter ist bevorzugt gasdurchlässig. Ein gasdurchlässiger Filter ermöglicht beispielsweise eine leichtere Befüllung des Reservoirs mit Wirkstoff, insbesondere bei der Herstellung der erfindungsgemäßen Vorrichtung. Hierzu kann der Wirkstoff bevorzugt mithilfe von Druckluft oder Vakuum in das Reservoir eingebracht werden, wobei der gasdurchlässige Filter den Durchtritt des Gases ermöglicht, aber den Wirkstoff im Reservoir zurückhält.

Der Filter kann beispielsweise die Form einer Scheibe aufweisen. Der Filter weist bevorzugt eine offene Porosität auf. In einigen Ausführungsformen weist der Filter offene Poren mit einer Porengröße kleiner 5 µm, bevorzugt kleiner 3 µm und besonders bevorzugt kleiner 1 µm auf.

Diese Porengröße kann durch den Ausschluss von sphärischen Partikeln mit einem entsprechenden Teilchendurchmesser bestimmt werden. Es handelt sich daher um nominelle Porengröße.

Der Filter kann durch eine Halterung innerhalb des Reservoirs befestigt sein. Die Halterung kann beispielsweise in Form eines Klemmrings ausgebildet sein. Gegebenenfalls kann der Filter in einer Aussparung in einer Wand des Reservoirs befestigt sein.

Der Filter kann beispielsweise Polyethylen, Polyurethan, Polyimid, Polyethersulfon, Polyvinylidenfluorid oder Poltetrafluorethylen aufweisen.

Der Filter kann ein Gewebefilter (woven filter) oder ein Vliesfilter (non-woven filter) sein.

In einigen Ausführungsformen umfasst das Reservoir einen zweiten Filter, der das Reservoir in zwei voneinander getrennte Bereiche, also einen ersten Bereich und einen zweiten Bereich, unterteilt. Der zweite Filter kann durch eine zweite Halterung innerhalb des Reservoirs befestigt sein. Der erste Bereich kann einen ersten Wirkstoff enthalten, und der zweite Bereich kann einen zweiten Wirkstoff enthalten, welcher sich von dem ersten Wirkstoff unterscheidet. Der erste Wirkstoff und der zweite Wirkstoff befinden sich bevorzugt jeweils in fester Form, beispielsweise Pulverform. Eine solche Ausführungsform ermöglicht die Herstellung einer Wirkstofflösung mit zwei verschiedenen Wirkstoffen, ohne dass die beiden Wirkstoffe in fester Form während der Lagerung der Vorrichtung miteinander in Berührung kommen.

Das Reservoir kann derart dimensioniert sein, dass das Verhältnis der Volumina des Reservoirs und des Behälters in einem Verhältnis von 1:25 zu 1:500 liegt. Das Volumen des Behälters kann beispielsweise 250 ml, 500 ml oder 1000 ml betragen. Dementsprechend kann das Volumen des Reservoirs beispielsweise 0,5 bis 10 ml, 1 bis 20 ml, oder 2 bis 40 ml betragen.

Die Vorrichtung weist weiterhin ein Fluidikelement auf. Das Fluidikelement ist bevorzugt zur Aufnahme einer Flüssigkeit aus einem Behälter in das Reservoir eingerichtet. Das Fluidikelement kann alternativ oder zusätzlich zur Abgabe einer Flüssigkeit aus dem Reservoir eingerichtet sein. Das Fluidikelement ist bevorzugt zum aktiven Fördern einer Flüssigkeit eingerichtet. Das Fluidikelement kann beispielsweise einen Kolben zum Fördern einer Flüssigkeit aufweisen. Das Fluidikelement kann eine Spritze oder eine Pumpe aufweisen.

In einer Ausführungsform weist der Dorn einen hohlen Innenraum auf, der zur Aufnahme einer Flüssigkeit aus einem Behälter in das Reservoir eingerichtet ist. Hierbei kann der Dorn beispielsweise in der Form einer Hohlnadel ausgestaltet sein. Der Dorn kann beispielsweise aus Kunststoff bestehen, oder einen Kunststoff umfassen. Bevorzugt ist der Dorn ausreichend steif, um ein Septum eines handelsüblichen Behälters für physiologische Kochsalzlösung zu durchdringen, jedoch nicht so hart und fest, dass eine Verletzungsgefahr für medizinisches Personal besteht. In einer Ausführungsform ist der Dorn keine Metallnadel.

In einer Ausführungsform weist das Fluidikelement einen innerhalb des Reservoirs beweglich angeordneten Kolben auf, der das Reservoir flüssigkeitsdicht abschließt. Das Fluidikelement kann somit beispielsweise als Spritze oder Spritzenpumpe ausgeführt sein. Das Fluidikelement kann auch andere Arten von Mitteln zur Bewegung einer Flüssigkeit aufweisen, z.B. eine Membranpumpe, Peristaltikpumpe, Druckluftkartusche, oder ein Anschluss für eine externe Druck- oder Vakuumquelle.

In einer Ausführungsform weist die Vorrichtung ein erstes Bauteil und ein zweites Bauteil auf, welche lösbar miteinander verbindbar sind. Beispielsweise kann das erste Bauteil das Anschlusselement und den Dorn enthalten, und/oder das zweite Bauteil kann das Reservoir, den Filter und das Fluidikelement enthalten. Das erste Bauteil und das zweite Bauteil können durch ein Verbindungselement, bevorzugt ein formschlüssiges Verbindungselement, beispielsweise mithilfe eines Gewindes oder Schnapphakens, miteinander verbindbar sein. Ein bevorzugtes Gewinde ist ein Luer-Lock-Gewinde. Das erste Bauteil kann bevorzugt ein Außengewinde aufweisen, das mit einem Innengewinde des zweiten Bauteils verbindbar ist. Das zweite Bauteil kann ein Außengewinde aufweisen, das mit einem Innengewinde des ersten Bauteils verbindbar ist.

Das erste Bauteil kann in einer Ausführungsform dazu eingerichtet sein, fluidleitend mit einem Behälter verbunden zu sein, während das zweite Bauteil vom ersten Bauteil gelöst und gegebenenfalls gegen eine andere Vorrichtung ausgewechselt wird, beispielsweise gegen einen Verschluss oder eine Düse. Das erste Bauteil kann somit als eine Art multifunktionaler Schnellverbinder für andere Komponenten dienen. Das zweite Bauteil kann eine Spritze umfassen, sonders bevorzugt eine Luer-Lock-Spritze.

Wie oben dargestellt kann die Vorrichtung ein Verbindungselement umfassen, um entweder einzelne Teile der erfindungsgemäßen Vorrichtung, insbesondere das erste Bauteil und das zweite Bauteil, miteinander zu verbinden, oder die Vorrichtung mit weiteren Vorrichtungen zu verbinden, oder beides. Hierbei kann entweder ein einziges Verbindungselement dazu eingerichtet sein, alternativ mit einer Vielzahl unterschiedlicher Teile verbunden zu werden, oder es können mehrere Verbindungselemente an unterschiedlichen Positionen der Vorrichtung vorgesehen sein. Bevorzugt ist ein Verbindungselement ein formschlüssiges Verbindungselement. Ein Beispiel für ein solches Verbindungselement ist ein Gewinde. In einigen Ausführungsformen ist das Verbindungselement ein Luer-Lock-Gewinde.

In einer Ausführungsform weist die Vorrichtung ein Verbindungselement auf, mit dessen Hilfe das erste Bauteil mit dem zweiten Bauteil verbindbar ist. In einigen Ausführungsformen ist dasselbe Verbindungselement zusätzlich eingerichtet, die Vorrichtung mit einem Verschluss, einer Düse oder einer sonstigen Abgabevorrichtung zu verbinden. Alternativ können jeweils einzelne, voneinander verschiedenen Verbindungselemente für diese Zwecke vorgesehen sein.

In einer Ausführungsform ist die Vorrichtung vollständig sterilisiert. In einer Ausführungsform ist die Vorrichtung vollständig pyrogenfrei. Der Begriff "sterilisiert" bezieht sich darauf, dass die Vorrichtung einem Verfahren unterzogen worden ist, um Krankheitserreger zu entfernen oder abzutöten, sodass die Vorrichtung gemäß der üblichen Standards wie zum Beispiel der Norm DIN EN 556-1 medizinisch verwendet werden kann. Verfahren zur Sterilisation umfassen beispielsweise Autoklavierung, Bestrahlung mit Elektronenstrahlen oder Gammastrahlen, oder Behandlung mit desinfizierenden Substanzen wie zum Beispiel Ethylenoxid. In ähnlicher Weise bezieht sich der Begriff "pyrogenfrei" darauf, dass die Vorrichtung einer im Fachgebiet üblichen Behandlung unterzogen worden ist, um Pyrogene zu entfernen oder zu zersetzen. Ob die Vorrichtung pyrogenfrei ist, kann mithilfe des Limulus-Amöbozytenlysat (LAL)-Tests gemäß Ph. Eur. Kapitel 2.6.14 geprüft werden. Hierbei gelten jeweils die Normen bzw. Dokumente, die zum Prioritätstag der vorliegenden Anmeldung aktuell sind.

In einer Ausführungsform weist die Vorrichtung weiterhin eine Düse auf, welche zur Abgabe des Wirkstoffs in gelöster Form eingerichtet ist. Die Düse kann einen im Wesentlichen zylindrischen, an einem Ende spitz zulaufenden Hohlkörper aufweisen. Die Düse kann gegebenenfalls ein Gelenk umfassen, das durch seitliches Biegen einer Öffnung der Düse relativ zur gesamten Vorrichtung die Abgabe einer Flüssigkeit in unterschiedliche Richtungen erlaubt.

Bevorzugt ist die Düse lösbar fluidleitend mit dem Anschlusselement verbindbar. Dies kann über ein formschlüssiges Verbindungselement, beispielsweise ein Gewinde, bevorzugt ein Luer-Lock-Gewinde, ermöglicht sein.

Die Vorrichtung kann weiterhin einen Schild umfassen. Der Schild kann eingerichtet sein, Teile der Vorrichtung vor Spritzern der abgegebenen Flüssigkeit, insbesondere einer Wirkstofflösung, zu schützen. Der Schild kann an oder in räumlicher Nähe zu der Düse angeordnet sein. Der Schild kann die Form einer Scheibe aufweisen. Der Schild kann eine konkave Krümmung aufweisen, ähnlich einer Parabolantenne. Der Schild kann eine zentral angeordnete Ausnehmung aufweisen, die zur Anbringung des Schilds an einem Ausgang der Vorrichtung eingerichtet ist.

In einer Ausführungsform ist der in der Vorrichtung enthaltene Wirkstoff ein Antiseptikum. Der Wirkstoff bzw. das Antiseptikum kann beispielsweise ein antiseptisches Oxidationsmittel sein. In einer Ausführungsform ist das Antiseptikum ausgewählt aus der Gruppe bestehend aus Calciumhypochlorit, Magnesiumhypochlorit, Natriumhypochlorit, Natriumpercarbonat, Natriumperoxid, Calciumperoxid, Harnstoff-Peroxid, Triisocyanurchlorid, Natrium-N-chlor-4-methylbenzolsulfonamid, Natrium-N-chlorbenzolsulfonamid, Octenidin, Polyhexanid und Chlorhexidindigluconat. In einer Ausführungsform ist das Antiseptikum ein Hypochloritsalz.

In einer Ausführungsform liegt der Wirkstoff als Pulver mit einer mittleren Partikelgröße kleiner 300 µm, bevorzugt kleiner 200 µm und besonders bevorzugt kleiner 100 µm vor. Durch die kleine Partikelgröße kann eine schnelle Auflösung der Wirkstoffpartikel erzielt werden. Die Partikelgröße des Wirkstoffs kann bevorzugt oberhalb des Ausschlussvolumens des Filters liegen. Dieser mittlere Durchmesser der Partikel kann durch fraktionierte Siebung bestimmt werden, wie es im Fachgebiet üblich ist. In einer Ausführungsform liegt der Wirkstoff als zylinderförmiger Pressling oder als zylinderförmige erstarrte Schmelze vor.

In einer Ausführungsform weist das Anschlusselement eine erste Durchführung auf, welche zur Aufnahme einer Flüssigkeit aus einem Behälter in das Reservoir eingerichtet ist. Die erste Durchführung bildet bevorzugt eine fluidleitende Verbindung zwischen dem Anschlusselement, insbesondere dem Dorn, und dem Reservoir.

In einer Ausführungsform ist die Vorrichtung zur Abgabe einer Flüssigkeit aus einem Behälter eingerichtet. Hierbei ist bevorzugt die Abgabe in einem Zustand möglich, in welchem die Vorrichtung über die oben beschriebene erste Durchführung mit dem Reservoir fluidleitend verbunden ist. Beispielsweise kann mithilfe einer solchen Ausführungsform Flüssigkeit aus einem mit der Vorrichtung verbundenen Behälter zunächst in das Reservoir der Vorrichtung aufgenommen werden, der enthaltene Wirkstoff in der Flüssigkeit gelöst werden, und der gelöste Wirkstoff nachfolgend in den Behälter abgegeben werden, während die Vorrichtung mit dem Behälter verbunden ist. Anschließend kann der gelöste Wirkstoff über die Vorrichtung aus dem Behälter an einen Patienten abgegeben werden, während die Vorrichtung, insbesondere das erste Bauteil der Vorrichtung, weiterhin mit dem Behälter verbunden ist. Gegebenenfalls kann die Vorrichtung zur Abgabe des gelösten Wirkstoffs mit einer Düse oder einer anderen Abgabevorrichtung verbunden werden, wobei ggf. das zweite Bauteil der Vorrichtung gegen die Düse oder andere Abgabevorrichtung ausgewechselt werden kann. Die Flüssigkeit ist bevorzugt eine medizinisch verträgliche Flüssigkeit. Die Flüssigkeit ist bevorzugt zur inneren Anwendung geeignet.

In einer Ausführungsform ist das Reservoir durchlässig für Gammastrahlen und/oder Elektronenstrahlen. Hierdurch wird es ermöglicht, den Innenraum und den enthaltenen Wirkstoff der Vorrichtung mithilfe von Gammastrahlen und/oder Elektronenstrahlen zu sterilisieren, um eine vollständig sterilisierte Vorrichtung zu erhalten.

In einer Ausführungsform definieren der Dorn und das Reservoir gemeinsam eine Transferachse, wobei entlang dieser Transferachse eine Abgabe der Flüssigkeit aus dem Reservoir und/oder in das Reservoir erfolgen kann. In einer Ausführungsform definieren der Behälter und das Reservoir gemeinsam eine Transferachse, wobei entlang dieser Transferachse eine Abgabe der Flüssigkeit aus dem Reservoir und/oder eine Aufnahme in das Reservoir erfolgen kann.

Ein weiterer Aspekt betrifft ein Kit, aufweisend eine Vorrichtung gemäß einer der vorangehenden Ausführungsformen und einen Behälter mit einer medizinisch verträglichen Flüssigkeit, wobei die Vorrichtung lösbar mit dem Behälter verbindbar ist. In einer Ausführungsform sind die Bestandteile des Kits, nämlich die Vorrichtung und der Behälter, gemeinsam keimdicht verpackt. In einer Ausführungsform sind die Bestandteile des Kits jeweils vollständig sterilisiert. In einer Ausführungsform sind die Bestandteile des Kits jeweils vollständig pyrogenfrei. Das Kit kann weiterhin eine Abgabevorrichtung zur Abgabe einer Flüssigkeit enthalten, beispielsweise eine Düse oder Spülvorrichtung, beispielsweise eine Spülvorrichtung gemäß EP4035605A1, welche hiermit durch Bezugnahme vollständig mit aufgenommen ist. Die medizinisch verträgliche Flüssigkeit kann eine Infusionslösung oder medizinische Spüllösung umfassen. Die medizinisch verträgliche Flüssigkeit kann eine physiologische Kochsalzlösung, eine Ringer-Lösung, oder eine vergleichbare kochsalzhaltige Lösung umfassen. Die medizinisch verträgliche Flüssigkeit ist bevorzugt steril und/oder pyrogenfrei.

Ein weiterer Aspekt betrifft einen Wirkstoff zur Verwendung in einem medizinischen Behandlungsverfahren, wobei der Wirkstoff mithilfe einer hierin beschriebenen Vorrichtung unmittelbar vor der Verabreichung an einen Patienten in einer Flüssigkeit gelöst und anschließend in gelöster Form verabreicht wird. Der Wirkstoff kann ein Stoff sein, welcher eine pharmakologische Wirkung aufweist. Der Wirkstoff kann beispielsweise eine antimikrobielle Wirkung aufweisen.

Ein weiterer Aspekt betrifft ein medizinisches Behandlungsverfahren, wobei der Wirkstoff mithilfe einer hierin beschriebenen Vorrichtung unmittelbar vor der Verabreichung an einen Patienten wie hierin beschrieben in einer Flüssigkeit gelöst und anschließend in gelöster Form verabreicht wird. Die Flüssigkeit ist bevorzugt eine medizinisch verträgliche Flüssigkeit wie hierin beschrieben.

Ein weiterer Aspekt betrifft ein Verfahren zur Herstellung einer Wirkstofflösung, welches die nachfolgenden Schritte umfasst,
a) Aufnahme einer Flüssigkeit aus einem Behälter mithilfe einer hierin beschriebenen Vorrichtung in das Reservoir der Vorrichtung,
b) Mischen des Wirkstoffs innerhalb des Reservoirs der Vorrichtung mit der in Schritt a) aufgenommenen Flüssigkeit,
c) dadurch Erhalt einer Wirkstofflösung,
d) gegebenenfalls Abgabe der Wirkstofflösung in den Behälter.

Das Verfahren kann bevorzugt in der Reihenfolge a), b), c) oder a), b), c), d) durchgeführt werden. Das Verfahren kann weiterhin einen Schritt umfassen, in welchem die Wirkstofflösung aus der Vorrichtung oder aus dem Behälter abgegeben wird. Bevorzugt kann die Wirkstofflösung aus dem Behälter über die Vorrichtung abgegeben werden, wie hierin andernorts ausführlicher beschrieben.

Ein weiterer Aspekt betrifft eine Wirkstofflösung, die nach einem oben dargestellten Verfahren herstellbar ist.

Ein weiterer Aspekt betrifft eine Verwendung einer hierin beschriebenen Vorrichtung oder eines hierin beschriebenen Kits zur Herstellung einer Wirkstofflösung.

Ein weiterer Aspekt betrifft ein Kit zur Verwendung bei der lokalen antiseptischen Behandlung eines Patienten, wobei das Kit ein antiseptisches Oxidationsmittel und ein Reduktionsmittel umfasst, wobei das antiseptische Oxidationsmittel durch Reaktion mit dem Reduktionsmittel zersetzbar ist.

Das Oxidationsmittel und das Reduktionsmittel liegen hierbei bevorzugt getrennt voneinander vor, beispielsweise in vollständig voneinander getrennten Behältern, oder innerhalb eines Behälters in verschiedenen voneinander getrennten Kompartimenten des Behälters. Solche Kompartimente können beispielsweise durch eine poröse Membran voneinander getrennt sein. Das Kit umfasst das Oxidationsmittel und das Reduktionsmittel bevorzugt in einer Form, welche eine zeitlich getrennte Verabreichung von Oxidationsmittel und Reduktionsmittel erlaubt.

Eine lokale antiseptische Behandlung bedeutet hierin jede Behandlung, bei der eine Desinfektion von Gewebe unmittelbar am Patienten erfolgt. Eine Desinfektion umfasst die Behandlung mit einem antimikrobiellen Wirkstoff, zum Beispiel einem antiseptischen Oxidationsmittel.

Ein Oxidationsmittel ist eine Substanz, die in der Lage ist, Elektronen von anderen Substanzen zu akzeptieren und dabei selbst reduziert wird. Durch die Akzeptanz von Elektronen führt ein Oxidationsmittel zu einer Oxidation der reagierenden Substanzen. Wie hierin verwendet, kann ein antiseptisches Oxidationsmittel durch eine solche Oxidation eine antimikrobielle Wirkung ausüben. In einigen Ausführungsformen ist das erfindungsgemäße Oxidationsmittel in der Lage, Sauerstoff auf eine andere Substanz zu übertragen. Beispiele für erfindungsgemäße Oxidationsmittel umfassen hypochlorige Säure, Calciumhypochlorit, Magnesiumhypochlorit, Natriumhypochlorit, Natriumpercarbonat, Natriumperoxid, Calciumperoxid, Chlorit-lonen-haltige Lösungen und Harnstoff-Peroxid.

Ein Reduktionsmittel ist eine Substanz, die Elektronen an andere Substanzen abgeben kann und dabei selbst oxidiert wird. Durch die Abgabe von Elektronen führt ein Reduktionsmittel zu einer Reduktion der reagierenden Substanzen. Erfindungsgemäß ist das hierin beschriebene Reduktionsmittel dazu in der Lage, das erfindungsgemäße antiseptische Oxidationsmittel zu reduzieren. In einer Ausführungsform ist das Reduktionsmittel dazu in der Lage, das antiseptische Oxidationsmittel unter Bildung von Wasser zu zersetzen.

Erfindungsgemäß verliert das antiseptische Oxidationsmittel durch Reaktion mit dem Reduktionsmittel seine oxidierende Eigenschaft und die darauf beruhende antiseptische Wirkung. Beispielsweise kann ein Hypochloritsalz durch Reaktion mit einem Reduktionsmittel wie z.B. Natriumascorbat zu einem Chloridsalz reagieren.

Ein Grundkonzept der vorliegenden Erfindung besteht darin, oxidierende antiseptische Lösungen zur Keimreduktion zu verwenden und diese nach Beendigung der Einwirkzeit durch Einwirkung einer Lösung eines Reduktionsmittels unter Bildung ungiftiger, nicht oxidierender Folgeprodukte, bevorzugte Folgeprodukte sind dabei Wasser und Chlorid-Ionen, innerhalb weniger Sekunden zu inaktivieren. Dadurch kann eine Schädigung des zuvor behandelten humanen oder tierischen Gewebes durch verbleibende Reste des oxidierenden Antiseptikums besser vermieden werden, insbesondere auch bei höheren Konzentrationen des Antiseptikums. Dies ermöglicht den verträglichen Einsatz höher konzentrierter Lösungen oxidierender Antiseptika. Damit kann die antiseptische Wirkung insbesondere gegenüber in Biofilmen enthaltenen Mikroorganismen deutlich gesteigert werden, ohne dass nachfolgende toxische Schäden eintreten können. Bevorzugte oxidierende Antiseptika sind Hypochlorit-Ionen, hypochlorige Säure und Chlorit-Ionen. Diese können leicht zu Wasser und Chlorid-Ionen reduziert werden.

Ascorbinsäure und Ascorbat-Ionen reagieren mit Sauerstoff-abspaltenden Oxidationsmitteln unter Bildung von Dehydroascorbinsäure, wobei als weiteres Produkt Wasser entsteht. Ein Vorteil von Ascorbinsäure ist, dass Ascorbinsäure und Dehydroascorbinsäure ein Redoxpaar bilden, das natürlicherweise im humanen Organismus vorkommt. Dehydroascorbinsäure hat den weiteren Vorteil, dass sie gut wasserlöslich ist.

In einer Ausführungsform der Erfindung enthält das Kit eine erste Lösung mit Hypochlorit-Ionen und/oder hypochloriger Säure als Oxidationsmittel, und eine zweite Lösung mit Ascorbinsäure und/oder Ascorbat-Ionen als Reduktionsmittel. Hypochlorit-Ionen und hypochlorige Säure können mit Ascorbinsäure zu Chlorid-Ionen, Wasser und Dehydroascorbinsäure reagieren. Diese Reaktionsprodukte sind ungiftig und stellen körpereigene Bestandteile des humanen Organismus dar.

In einer zweiten Ausführungsform des Kits zur Verwendung gemäß dieses Aspekts umfasst die Verwendung ein Inkontaktbringen des Oxidationsmittels mit einer Gewebeoberfläche des Patienten und ein nachfolgendes Inkontaktbringen des Reduktionsmittels mit der Gewebeoberfläche des Patienten.

In einer Ausführungsform kann demnach ein Patient, insbesondere eine Gewebeoberfläche des Patienten, zur lokalen antiseptischen Behandlung zunächst mit einer ersten Lösung in Kontakt gebracht werden, welche ein antiseptisches Oxidationsmittel enthält. Anschließend kann eine zweite Lösung an derselben Stelle in Kontakt mit dem Patienten gebracht werden, um Reste der ersten Lösung wie oben dargestellt zu inaktivieren. Gegebenenfalls können die erste Lösung und/oder die zweite Lösung nach der Behandlung oder nach Aufbringen der ersten Lösung entfernt werden, beispielsweise durch Absaugen oder Abtupfen der behandelten Gewebeoberfläche des Patienten. Hierdurch können Mikroorganismenreste, Biofilmreste und sonstige Debris vom Patienten entfernt werden.

Diese erste Lösung kann gegebenenfalls frisch hergestellt werden, beispielsweise wie es hierin ausführlich auch im Zusammenhang mit der erfindungsgemäßen Vorrichtung beschrieben ist. Hierzu kann ein antiseptisches Oxidationsmittel, welches in fester Form vorliegt, vom behandelnden Arzt in einem wässrigen Medium gelöst werden. Dies kann bevorzugt unmittelbar vor der Behandlung des Patienten geschehen. In entsprechender Weise kann auch die zweite Lösung gegebenenfalls unmittelbar vor der Behandlung in derselben Weise hergestellt werden, indem das Reduktionsmittel in Wasser oder einer wässrigen Lösung gelöst wird. Gegebenenfalls können bei diesen Vorgängen weitere Hilfsstoffe wie zum Beispiel Puffersubstanzen und/oder Tenside hinzugefügt werden. Alternativ können diese Hilfsstoffe bereits in einem wässrigen Medium vorliegen, welches mit dem Oxidationsmittel bzw. dem Reduktionsmittel zu einer fertigen Lösung gemischt wird.

Durch die Herstellung der Lösungen unmittelbar vor deren Verwendung können unerwünschte Zersetzungsreaktionen des Oxidationsmittels bzw. des Reduktionsmittels vermieden werden.

In einigen Ausführungsformen werden das antiseptische Oxidationsmittel und/oder das Reduktionsmittel daher in fester Form bereitgestellt, um eine solche frische Herstellung der jeweiligen Lösungen zu ermöglichen. In einer Ausführungsform liegt das antiseptische Oxidationsmittel als Feststoff vor, und das Reduktionsmittel liegt als Lösung vor. In einer Ausführungsform liegt das antiseptische Oxidationsmittel als Lösung vor, und das Reduktionsmittel liegt als Feststoff vor. In einer Ausführungsform liegen sowohl das antiseptische Oxidationsmittel als auch das Reduktionsmittel als Feststoff vor. In einer Ausführungsform liegen sowohl das antiseptische Oxidationsmittel als auch das Reduktionsmittel als Lösung vor.

Für die antiseptische Behandlung ist es bei vielen Anwendungsbereichen bevorzugt, beispielsweise bei der Behandlung von offen liegenden künstlichen Gelenken und offen liegendem Weich- und Knochengewebe, sterile Wirkstoffe bzw. Lösungen einzusetzen. Daher werden in einigen Ausführungsformen das Oxidationsmittel und/oder das Reduktionsmittel, gegebenenfalls als Feststoff oder als Lösung, jeweils in steriler Form bereitgestellt.

In einer Ausführungsform enthält das Kit das antiseptische Oxidationsmittel als sterilen Feststoff. In einer Ausführungsform enthält das Kit das Reduktionsmittel als sterilen Feststoff. In einer Ausführungsform enthält das Kit weiterhin ein oder mehrere sterile Flüssigkeiten, beispielsweise Wasser für Injektionszwecke, sterile Kochsalzlösung, Ringer-Lösung oder andere sterile wässrige Komponenten.

In einer Ausführungsform enthält das Kit eine sterile erste Lösung mit einem Oxidationsmittel und/oder eine sterile zweite Lösung mit einem Reduktionsmittel. Das Kit kann weiterhin Puffersubstanzen und/oder weitere Hilfsstoffe in steriler Form umfassen, entweder als Feststoffe oder als wässrige Lösungen.

Bevorzugt werden eine erste sterile Lösung mit einem antiseptischen Oxidationsmittel und eine zweite sterile Lösung mit einem Reduktionsmittel unmittelbar vor ihrer Verwendung durch Auflösung eines sterilen antiseptischen Oxidationsmittels in fester Form und Auflösung eines sterilen Reduktionsmittels in fester Form hergestellt.

In einer Ausführungsform kann die erste Lösung mithilfe einer hierin beschriebenen Vorrichtung auf eine Gewebeoberfläche des Patienten aufgebracht werden. Alternativ oder zusätzlich kann die zweite Lösung mithilfe einer hierin beschriebenen Vorrichtung auf eine Gewebeoberfläche des Patienten aufgebracht werden. Die erste Lösung und/oder die zweite Lösung können grundsätzlich durch jedes geeignete Mittel auf die Gewebeoberfläche aufgebracht werden, beispielsweise durch Sprühen mithilfe von Spritzflaschen oder motorgetriebenen Lavage-Systemen. Nach der Behandlung können die erste Lösung und/oder die zweite Lösung durch Absaugen oder eine andere geeignete Aufnahme von der Gewebeoberfläche entfernt werden, beispielsweise mithilfe eines Tupfers.

In einer dritten Ausführungsform des Kits zur Verwendung gemäß dieses Aspekts erfolgt das Inkontaktbringen des Oxidationsmittels und/oder des Reduktionsmittels mit der Gewebeoberfläche des Patienten jeweils für eine Zeitdauer von 10 Sekunden bis 300 Sekunden, beispielsweise 10 bis 20 Sekunden, 10 bis 30 Sekunden, 10 bis 50 Sekunden, 10 bis 60 Sekunden, oder 10 bis 120 Sekunden.

In einer vierten Ausführungsform des Kits zur Verwendung gemäß dieses Aspekts umfasst das Kit eine Stoffmenge des Reduktionsmittels, welche mindestens der Stoffmenge des Oxidationsmittels entspricht. In einer Ausführungsform umfasst das Kit eine ausreichende Menge Reduktionsmittel, um das im Kit enthaltene Oxidationsmittel vollständig zu neutralisieren.

In einer fünften Ausführungsform des Kits zur Verwendung gemäß dieses Aspekts ist das Oxidationsmittel ausgewählt aus der Gruppe bestehend aus Natriumhypochlorit (CAS 7681-52-9), Calciumhypochlorit (CAS 7778-54-3) und Natriumchlorit (CAS 7758-19-2).

Bevorzugte Reduktionsmittel sind medizinisch verträgliche Substanzen, welche mit hypochloriger Säure und/oder Hypochloritsalzen in einer Redoxreaktion zu Chloridsalzen und Wasser reagieren. In einer sechsten Ausführungsform des Kits zur Verwendung gemäß dieses Aspekts ist das Reduktionsmittel ausgewählt aus der Gruppe bestehend aus Ascorbinsäure (CAS 50-81-7), Natriumascorbat (CAS 134-03-2), Kaliumascorbat (CAS 15421-15-5), Calciumascorbat (CAS 5743-27-1), L-Cystin (CAS 52-90-4) und Cysteamin (CAS 60-23-1).

In einer siebten Ausführungsform des Kits zur Verwendung gemäß dieses Aspekts umfasst das Kit weiterhin eine Puffersubstanz, wobei die Puffersubstanz bevorzugt ausgewählt ist aus der Gruppe bestehend aus Citronensäure (CAS 77-92-9), Natriumdihydrogencitrat (CAS 18996-35-5), Dinatriumhydrogencitrat (CAS 6132-05-4), Natriumdihydrogenphosphat (CAS 7558-80-7), Calciumdihydrogenphosphat (CAS 7758-23-8), Natriumhydrogensulfat (CAS 7681-38-1), Dinatriumhydrogenphosphat (CAS 7558-79-4) und Trinatriumphosphat (CAS 760-54-9), besonders bevorzugt Citronensäure. Besonders bevorzugte Puffersubstanzen sind medizinisch verträgliche Stoffe, welche mit den übrigen Komponenten des Kits, insbesondere dem antiseptischen Oxidationsmittel und/oder dem Reduktionsmittel, eine klare Lösung bilden können.

Beispielsweise hat Citronensäure den besonderen Vorteil, dass sie mit Calcium-Ionen einen wasserlöslichen Komplex bilden kann. Dadurch können die mit Calciumhypochlorit und Citronensäure gebildeten Lösungen vollständig sichtklar sein.

In einer achten Ausführungsform des Kits zur Verwendung gemäß dieses Aspekts umfasst das Kit eine erste Lösung mit Hypochlorit-Ionen, Chloritionen und/oder hypochloriger Säure in einer Konzentration von insgesamt (d.h. kumulativ) 50 ppm bis 5000 ppm. In einer Ausführungsform enthält die erste Lösung 50 ppm bis 5000 ppm Hypochlorit-Ionen. In einer Ausführungsform enthält die erste Lösung 50 ppm bis 5000 ppm Chlorit-Ionen. In einer Ausführungsform enthält die erste Lösung 50 ppm bis 5000 ppm hypochlorige Säure. In einer Ausführungsform enthält die erste Lösung insgesamt 50 ppm bis 5000 ppm dieser drei genannten Spezies, nämlich Hypochlorit-Ionen, Chloritionen und hypochlorige Säure.

In einer Ausführungsform umfasst das Kit eine erste Lösung, welche Hypochlorit-Ionen, Chlorit-Ionen und/oder hypochlorige Säure in einer Konzentration von 50 ppm bis 5000 ppm enthält. Chlorit-Ionen sind starke Oxidationsmittel und haben eine starke antiseptische Wirkung.

Die Maßeinheit ppm bedeutet "parts per million"(Teile pro Million), und gibt beispielsweise an, wie viele Teile Hypochlorit pro eine Million Teile der Lösung vorhanden sind, basierend auf dem Gewicht der jeweiligen Komponenten, und bezogen auf das Gesamtgewicht der Lösung. Es wird mit der Einheit ppm also jeweils der Massenanteil eines Stoffs, beispielsweise der Hypochlorit-Ionen, in der Lösung ausgedrückt. Die Maßeinheit ppm bezieht sich daher hierin stets auf einen Massenanteil, und nicht auf einen Stoffmengenanteil.

In einer neunten Ausführungsform des Kits zur Verwendung gemäß dieses Aspekts umfasst das Kit eine zweite Lösung mit Ascorbinsäure und/oder Ascorbat-Ionen in einer Konzentration von 50 ppm bis 10.000 ppm.

In einer zehnten Ausführungsform des Kits zur Verwendung gemäß dieses Aspekts umfasst das Kit eine erste Lösung mit dem Oxidationsmittel und/oder eine zweite Lösung mit einem Reduktionsmittel mit einer Osmolarität von jeweils mindestens 310 mOsmol/L. Das bedeutet, dass die erste Lösung und die zweite Lösung bevorzugt isotonisch oder hypertonisch sind. Dadurch kann eine hypotone Schädigung von humanen oder tierischen Zellen durch die Lösungen vermieden werden.

In einer elften Ausführungsform des Kits zur Verwendung gemäß dieses Aspekts umfasst das Kit eine erste Lösung mit einem Oxidationsmittel und einem pH-Wert im Bereich von pH 4 bis pH 8. In diesem pH-Bereich liegt das Gleichgewicht zwischen Hypochlorit-Ionen und hypochloriger Säure auf der Seite der hypochlorigen Säure. Die hypochlorige Säure hat eine höhere antimikrobielle Aktivität als Hypochlorit-Ionen.

In einer zwölften Ausführungsform des Kits zur Verwendung gemäß dieses Aspekts umfasst das Kit eine zweite Lösung mit einem Reduktionsmittel und einem pH-Wert im Bereich von pH 4 bis pH 7. In einer Ausführungsform umfasst das Kit zur Verwendung gemäß dieses Aspekts erste Lösung mit einem Oxidationsmittel und einem pH-Wert im Bereich von pH 4 bis pH 8 und eine zweite Lösung mit einem Reduktionsmittel und einem pH-Wert im Bereich von pH 4 bis pH 7.

In einer dreizehnten Ausführungsform des Kits zur Verwendung gemäß dieses Aspekts wird/werden eine erste Lösung mit dem Oxidationsmittel und/oder eine zweite Lösung mit dem Reduktionsmittel jeweils unmittelbar vor dem Inkontaktbringen mit der Gewebeoberfläche durch Lösen des Oxidationsmittels bzw. Reduktionsmittels in fester Form, gegebenenfalls gemeinsam mit einer Puffersubstanz, in Wasser hergestellt.

In einer vierzehnten Ausführungsform des Kits zur Verwendung gemäß dieses Aspekts umfasst die Verwendung die Behandlung eines Krankheitszustands, der ausgewählt ist aus der Gruppe bestehend aus
a) freiliegende Oberflächen von künstlichen Gelenken und gelenknahen Knochen- und Weichgebe, bevorzugt im Rahmen von DAIR-Prozeduren,
b) Wunden mit freiliegendem Knochengewebe, Knorpelgewebe, Bändern und Sehnen,
c) Intra- und postoperative Operationswunden,
d) chronischen Wunden,
e) Dekubiti,
f) Diabetische Ulcera,
g) Ulcus cruris,
h) Bisswunden,
i) Schnittwunden,
j) Stichwunden,
k) Schnittwunden,
l) Risswunden,
m) Schürfwunden,
n) Schussverletzungen,
o) Splitterverletzungen,
p) Fisteln,
q) Abszesse und
r) Verbrennungen ersten und zweiten Grades.

Unter dem Begriff der DAIR-Prozedur (DAIR: Debridement, Antibiotics, Implant retention) wird eine offene Prothesenrevision unter weitgehendem Erhalt der Gelenkprothese verstanden.

In einer fünfzehnten Ausführungsform des Kits zur Verwendung gemäß dieses Aspekts umfasst das Kit weiterhin ein Tensid, wobei die Verwendung das Inkontaktbringen des Tensids gemeinsam mit dem Oxidationsmittel mit einer Gewebeoberfläche des Patienten umfasst, wobei das Tensid bevorzugt ein Alkylsulfat ist. Ein bevorzugtes Alkylsulfat ist Natriumdodecylsulfat. Tenside können die Wirkung des antiseptischen Oxidationsmittels auf Biofilme verbessern, indem sie die Biofilme destabilisieren und somit die Zugänglichkeit für das antiseptische Oxidationsmittel erhöhen.

Das Kit kann eine hierin dargestellte Vorrichtung zur Herstellung einer Wirkstofflösung umfassen. Bevorzugt weist diese Vorrichtung die folgenden Bestandteile auf:
- ein Anschlusselement, das zum flüssigkeitsdichten Anschluss an einen Behälter ausgebildet und eingerichtet ist;
- einen Dorn, der zum Durchstechen eines Septums eines Behälters ausgebildet und eingerichtet ist;
- ein Reservoir, das einen Wirkstoff in fester Form enthält, und über einen Ausgang des Reservoirs fluidleitend mit dem Anschlusselement verbindbar ist;
- einen flüssigkeitsdurchlässigen Filter, der an dem Ausgang des Reservoirs angeordnet ist, und eingerichtet ist, den Wirkstoff in fester Form in dem Reservoir zurückzuhalten;
- ein Fluidikelement, welches zur Aufnahme einer Flüssigkeit aus einem Behälter in das Reservoir eingerichtet ist, und/oder zur Abgabe einer Flüssigkeit aus dem Reservoir eingerichtet ist.

Die Vorrichtung ist bevorzugt ausgebildet und eingerichtet, eine Flüssigkeit aus einem Behälter in das Reservoir aufzunehmen, den Wirkstoff in der Flüssigkeit zu lösen, und den gelösten Wirkstoff über das Anschlusselement an einen Patienten abzugeben.

Der Wirkstoff ist bevorzugt ein antiseptisches Oxidationsmittel.

Die Vorrichtung weist bevorzugt ein Reduktionsmittel auf, welches das Oxidationsmittel durch eine Redoxreaktion neutralisieren kann.

Das Kit kann zwei solche Vorrichtungen umfassen, wobei eine der Vorrichtungen ein antiseptisches Oxidationsmittel umfasst, und die andere der beiden Vorrichtungen ein Reduktionsmittel umfasst.

Ein weiterer Aspekt der Erfindung betrifft ein Therapieverfahren, wobei das Therapieverfahren ein Inkontaktbringen eines antiseptischen Oxidationsmittels mit einer Gewebeoberfläche eines Patienten und ein nachfolgendes Inkontaktbringen eines Reduktionsmittels mit der Gewebeoberfläche des Patienten umfasst. Die Ausführungsformen des hierin beschriebenen Kits zur Verwendung bei der lokalen antiseptischen Behandlung eines Patienten können für dieses Therapieverfahren in entsprechender Weise Anwendung finden.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

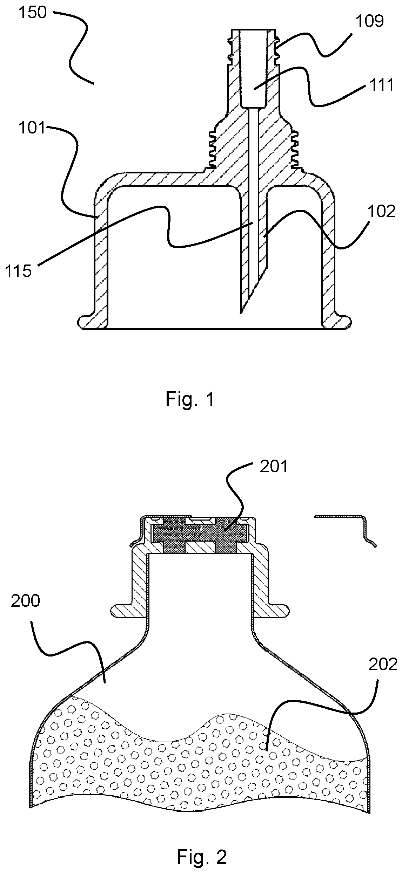

**Figur 1** zeigt beispielhaft ein erstes Bauteil **150** einer Vorrichtung **100,** welches mit einem Behälter **200** verbindbar ist. Das erste Bauteil **150** der Vorrichtung weist einen Anschlusselement **101** auf, welches formschlüssig mit einem Behälter verbunden werden kann, beispielsweise mit einem dafür vorgesehenen Anschluss am Kopf einer Infusionsflasche oder eines Folienbeutels. Die Vorrichtung weist weiterhin einen Dorn **102** auf, mit dem ein Septum **201** eines Behälters durchstochen werden kann. Der Dorn **102** weist einen inneren Hohlraum **115** auf, um eine Flüssigkeit aus dem Behälter in die Vorrichtung zu leiten. Der innere Hohlraum ist mit einer Durchführung **111** der Vorrichtung fluidleitend verbunden. Das erste Bauteil **150** der Vorrichtung **100** weist weiterhin ein Verbindungselement **109** auf, mit dem das erste Bauteil **150** der Vorrichtung mit einem zweiten Bauteil **160** der Vorrichtung verbindbar ist. Das Verbindungselement **109** ist in diesem Beispiel als Luer-Lock-Gewinde ausgeführt.
**Figur 2** zeigt einen Behälter **200,** der mit einer erfindungsgemäßen Vorrichtung verbunden werden kann. Der Behälter **200** enthält eine medizinisch verträgliche Flüssigkeit **202,** und weist ein Septum **201** auf, welches den Behälter nach außen abdichtet. Das Septum **201** ist aus einem elastischen Material, beispielsweise Silikon, gebildet. Das Septum kann zusätzlich durch ein Siegel geschützt sein, das vor der Verbindung mit der erfindungsgemäßen Vorrichtung entfernt werden kann.

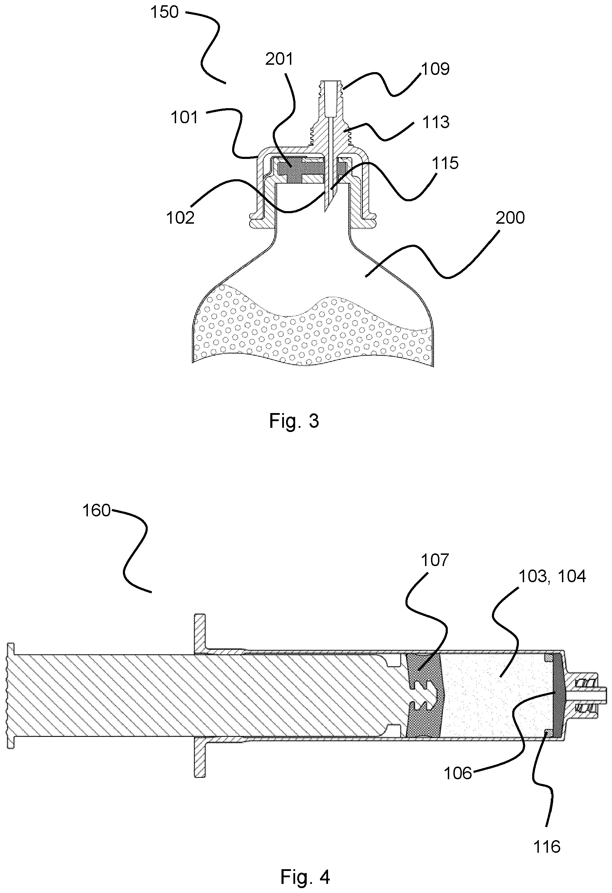

**Figur 3** zeigt ein erstes Bauteil **150** einer erfindungsgemäßen Vorrichtung, welche an einen Behälter **200** angebracht ist. Der Dorn **102** durchdringt das Septum **201** des Behälters **200,** sodass die Flüssigkeit **202** aus dem Behälter durch den Hohlraum **115** und die erste Durchführung **109** in die Vorrichtung überführt werden kann. Die Vorrichtung umfasst ein erstes Gewinde **109** und ein zweites Gewinde **113.** Beide Gewinde (**109, 113**) des ersten Bauteils **150** sind hier als Außengewinde ausgestaltet.
**Figur 4** zeigt einen zweiten Teil **160** einer erfindungsgemäßen Vorrichtung **100** mit einem Reservoir **103** für einen Wirkstoff **104** und einem Fluidikelement **107** zum Transport einer Flüssigkeit **202** aus einem Behälter **200.** Das Fluidikelement **107** ist hier als Spritze ausgeführt. Der Wirkstoff **104** ist als festes Pulver in dem Reservoir **103** angeordnet. Die Vorrichtung umfasst weiterhin einen Filter **106,** um den Wirkstoff **104** in dem Reservoir **103** zu halten. Der Wirkstoff **104** kann den Filter **106** nur in gelöster Form passieren. Der Filter **106** ist durch eine Halterung **116** in dem Reservoir **103** befestigt, um das Reservoir **103** nach außen abzudichten, sodass kein fester Wirkstoff **104** durch den Filter **106** aus dem Reservoir **103** gelangen kann. Das zweite Bauteil **160** weist ein Verbindungselement zum Anschluss an das erste Bauteil **150** auf. Dieses Verbindungselement des zweiten Bauteils **160** ist komplementär zu dem Verbindungselement des ersten Bauteils **150** ausgestaltet. Im hier gezeigten Beispiel weist das zweite Bauteil **160** ein Luer-Lock-Innengewinde auf.

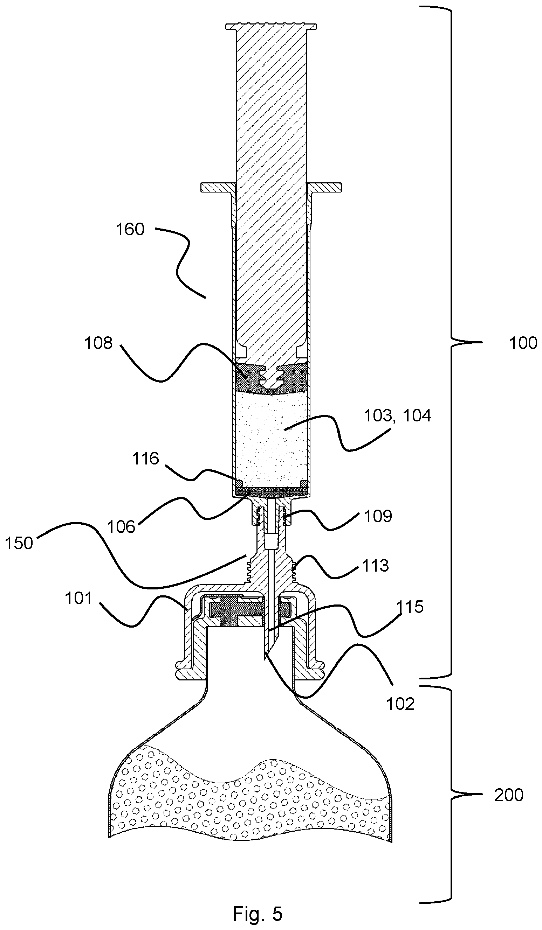

**Figur 5** zeigt eine erfindungsgemäße Vorrichtung **100,** die mit einem Behälter **200** verbunden ist. Das erste Bauteil **150** der Vorrichtung ist über ein Verbindungselement **109** mit dem zweiten Bauteil **160** der Vorrichtung fluidleitend und flüssigkeitsdicht verbunden. Das Anschlusselement **101** ist mit einem Behälter **200** verbunden, wobei der Dorn **102** durch ein Septum des Behälters in den Behälter eingeführt ist. Der Innenraum des Behälters ist durch einen Hohlraum **115** des Dorns mit dem Reservoir **103** fluidleitend verbunden. Mithilfe eines Fluidikelement, das hier einen Kolben **108** aufweist, kann nun eine Flüssigkeit aus dem Behälter **200** in das Reservoir **103** geleitet werden.

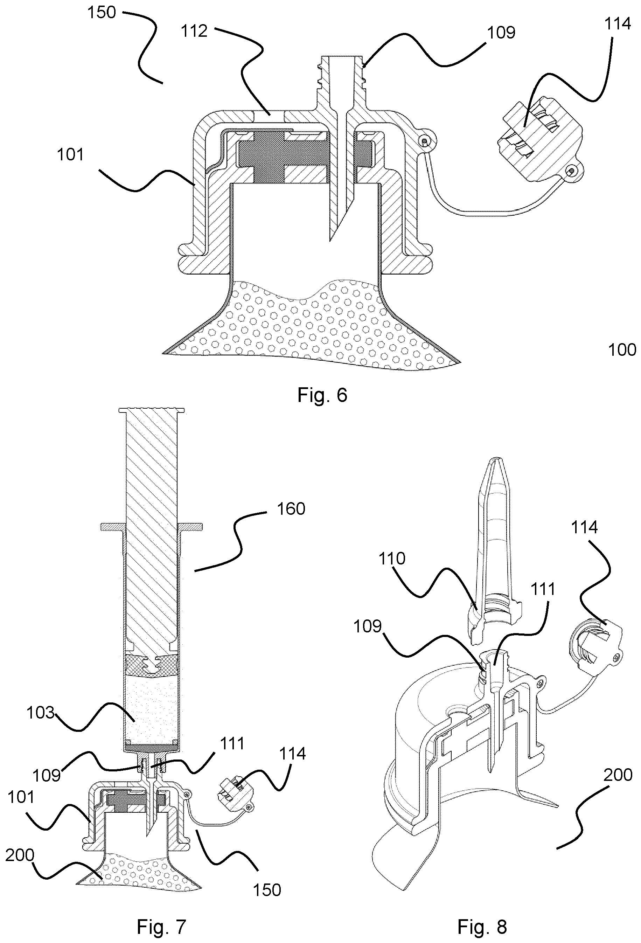

**Figur 6** zeigt ein erstes Bauteil **150** einer erfindungsgemäßen Vorrichtung, welches ein Verbindungselement **109** zur Verbindung mit einem Verschluss **114** oder einem zweiten Bauteil der Vorrichtung **160** aufweist. In der hier gezeigten Ausführungsform ist das als Gewinde ausgeführte Verbindungselement **109** alternativ mit verschiedenen Elementen verbindbar. Das Gewinde **109** kann entweder mit dem zweiten Bauteil der Vorrichtung **160** fluidleitend verbunden werden, oder mit einem Verschluss **114** flüssigkeitsdicht verschlossen werden.
**Figur 7** zeigt ein erstes Bauteil **150** einer erfindungsgemäßen Vorrichtung gemäß Figur 6, der über das Gewinde **109** mit einem zweiten Bauteil **160** der Vorrichtung verbunden ist. In dieser Konfiguration kann durch Zusammenwirken der beiden Bauteile **150** und **160** der Vorrichtung Flüssigkeit aus dem Behälter **200** entnommen werden. Die Flüssigkeit kann aus dem Behälter **200** über eine erste Durchführung **111** in das Reservoir **103** gepumpt werden. Innerhalb des Reservoirs **103** kann mithilfe der Flüssigkeit der im Reservoir **103** enthaltene Wirkstoff **104** gelöst werden, und die somit erhaltene Wirkstofflösung durch den Filter **106** in den Behälter **200** geleitet werden. Danach kann das zweite Bauteil **160** der Vorrichtung entfernt werden, und die Wirkstofflösung kann mithilfe des ersten Bauteils **150** der Vorrichtung aus dem Behälter abgegeben werden, beispielsweise an einen Patienten abgegeben werden.
**Figur 8** zeigt ein erstes Bauteil **150** einer erfindungsgemäßen Vorrichtung gemäß Figur 6, der über ein Gewinde **109** mit einer Düse **110** verbindbar ist. Mithilfe der Düse **110** und der Durchführung **111** kann die mithilfe der Vorrichtung hergestellte Wirkstofflösung aus dem Behälter **200** abgegeben werden. Gegebenenfalls kann die Durchführung **111** durch Anbringen eines Verschlusses **114** an dem Verbindungselement **109** flüssigkeitsdicht verschlossen werden.

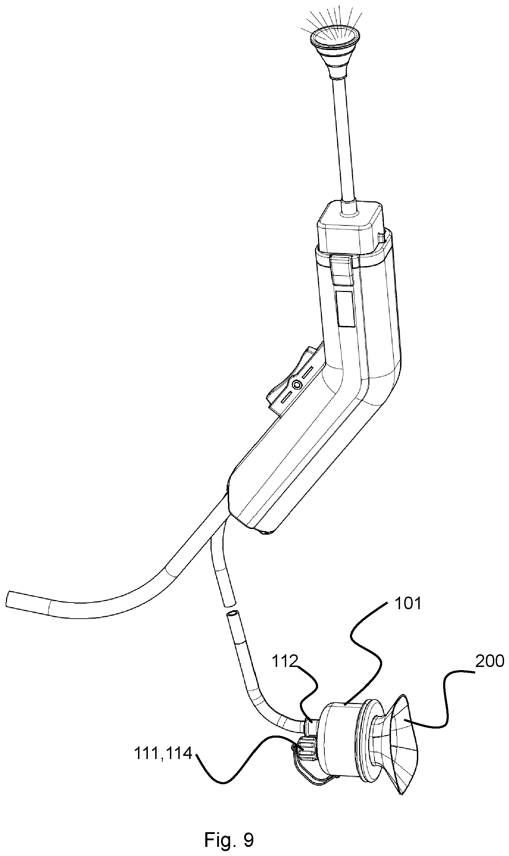

**Figur 9** zeigt eine erfindungsgemäße Vorrichtung, die gleichzeitig mit einem Behälter **200** und mit einer externen Abgabevorrichtung verbunden ist. Hierzu umfasst die Vorrichtung neben einer ersten Durchführung **111,** die hier durch einen Verschluss **114** flüssigkeitsdicht verschlossen ist, eine zweite Durchführung **112,** die zum Anschluss an eine externe Abgabevorrichtung eingerichtet ist. In der hier gezeigten Konfiguration ist das erste Bauteil **150** mithilfe des Anschlusselements **101** mit einem Behälter **200** fluidleitend verbunden, sodass eine mithilfe der Vorrichtung hergestellte Wirkstofflösung aus dem Behälter **200** über die zweite Durchführung **112** abgegeben werden kann.

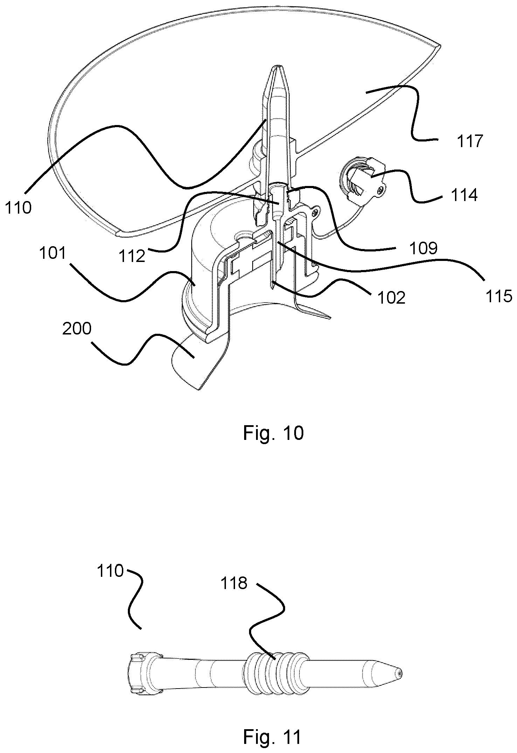

**Figur 10** zeigt ein erstes Bauteil einer Vorrichtung, die mit einer Düse **110** und einem Schild **117** versehen ist. Das erste Bauteil ist zur Abgabe einer Wirkstofflösung aus dem Behälter **200** eingerichtet. Das Schild **117** besitzt im hier gezeigten Beispiel eine zentrale, zylinderförmige Ausnehmung zur Befestigung an dem ersten Bauteil **150** und/oder der Düse **110.** Das Schild kann als Spritzschutz dienen, wenn eine Wirkstofflösung mithilfe der Düse **110** abgegeben wird.
**Figur 11** zeigt eine Düse **110** mit einem Gelenk **118,** die mit einer erfindungsgemäßen Vorrichtung verwendbar ist. Mithilfe des Gelenks **118** kann die Düse **110** gebogen werden, sodass die Austrittsöffnung der Düse einen Winkel mit dem Anschluss der Düse bildet, der sich von 180° unterscheidet. Somit kann eine Wirkstofflösung in verschiedene Richtungen abgegeben werden, ohne die gesamte Vorrichtung zu drehen.

Für die nachfolgenden Ausführungsbeispiele wurden Natriumchlorid (Sigma-Aldrich), Calciumhypochlorit (Sigma-Aldrich), Citronensäure (Sigma-Sldrich), Natriumchlorit (Sigma-Aldrich), Ascorbinsäure (Sigma-Aldrich) und Natriumascorbat (Sigma-Aldrich) sowie dest. Wasser verwendet.

### BEISPIELE 1-22

Die nachfolgenden Lösungen A, B und C wurden mit Hilfe von Magnetrührern in 1000 ml Erlenmeyerkolben angesetzt. Durch das in einer Konzentration von 0,9 % (Gew.) gelöste Natriumchlorid wird sichergestellt, dass alle Lösungen A, B und C eine Osmolarität größer 310 mOsmol haben.

| | **Lösung A** | | | | |
|---|---|---|---|---|---|
| **Beispiel Nr.** | **Dest. Wasser [ml]** | **NaCl [g]** | **Ca(OCl)₂ [g]** | | **Citronensäure [g]** |
| | | | **[g]** | **ppm (OCl⁻)** | |
| A1 | 500 | 0,45 | 0,143 | 100 | 0,143 |
| A2 | 500 | 0,45 | 0,286 | 200 | 0,286 |
| A3 | 500 | 0,45 | 0,572 | 400 | 0,572 |
| A4 | 500 | 0,45 | 0,715 | 500 | 0,715 |
| A5 | 500 | 0,45 | 1,430 | 1000 | 1,430 |
| A6 | 500 | 0,45 | 2,860 | 2000 | 2,860 |
| A7 | 500 | 0,45 | 7,150 | 5000 | 7,150 |

| | **Lösung B** | | | | |
|---|---|---|---|---|---|
| **Beispiel Nr.** | **Dest. Wasser [ml]** | **NaCl [g]** | **NaClO₂ [g]** | | **Citronensäure [g]** |
| | | | **[g]** | **ppm (ClO₂⁻)** | |
| B1 | 500 | 0,45 | 0,134 | 100 | 0,213 |
| B2 | 500 | 0,45 | 0,268 | 200 | 0,426 |

Die Lösungen A1-A7 und die Lösungen B1 und B2 hatten einen mit einer pH-Elektrode gemessenen pH-Wert zwischen pH 4,7 und 5,0.

| **Beispiel Nr.** | **Lösung C** | | | |
|---|---|---|---|---|
| | **Dest. Wasser [g]** | **NaCl [g]** | **Natriumascorbat [g]** | **Ascorbinsäure [g]** |
| C1 | 500 | 0,45 | 0,700 | 0,062 |
| C2 | 500 | 0,45 | 1,400 | 0,122 |
| C3 | 500 | 0,45 | 2,800 | 0,248 |
| C4 | 500 | 0,45 | 3,500 | 0,310 |
| C5 | 500 | 0,45 | 7,000 | 0,620 |
| C6 | 500 | 0,45 | 14,000 | 1,240 |
| C7 | 500 | 0,45 | 35,00 | 3,10 |

Die Lösungen C1-C7 hatten einen mit einer pH-Elektrode gemessenen pH-Wert bei 5,3-5,5.

Zur Prüfung der neutralisierenden Wirkung der Lösungen C wurden jeweils 20 ml der Lösungen A1-A7, B1 und B2 mit jeweils 20 ml der Lösungen C1-C7 in einem Becherglas mit einem Magnetrührer vermischt. Nach zwei Minuten wurde 2,0%ige Kaliumjod-Lösung und 1%ige Stärke dazugegeben und beobachtet, ob ein Farbumschlag nach blauschwarz infolge der Oxidation von Jodid zu Jod eintrat. Ein Farbumschlag nach blauschwarz zeigt die Anwesenheit von nicht zersetzten Hypochlorit-Ionen oder Chlorit-Ionen an, welche in der Lage sind, Jodid-Ionen zu Jod oxidieren. Das durch Oxidation der Jodid-Ionen entstandene Jod bildet mit Stärke einen blauschwarzen Komplex. Die Lösungen A1 bis A7, B1 und B2 wurden zuerst auf ihre Fähigkeit zur Oxidation von Jodid-Ionen zu Jod geprüft.

| **Versuch** | **Lösung A** | **Lösung C** | **Beobachtung** |
|---|---|---|---|
| 1 | A1 | - | blauschwarz |
| 2 | A2 | - | blauschwarz |
| 3 | A3 | - | blauschwarz |
| 4 | A4 | - | blauschwarz |
| 5 | A5 | - | blauschwarz |
| 6 | A6 | - | blauschwarz |
| 7 | A7 | - | blauschwarz |
| 8 | A1 | C1 | farblos |
| 9 | A2 | C2 | farblos |
| 10 | A3 | C3 | farblos |
| 11 | A4 | C4 | farblos |
| 12 | A5 | C5 | farblos |
| 13 | A6 | C6 | farblos |
| 14 | A7 | C7 | farblos |
| 15 | A1 | C2 | farblos |
| 16 | A2 | C3 | farblos |
| 17 | A3 | C4 | farblos |
| 18 | A4 | C5 | farblos |

| **Versuch** | **Lösung B** | **Lösung C** | **Beobachtung** |
|---|---|---|---|
| 19 | B1 | - | blauschwarz |
| 20 | B2 | - | blauschwarz |
| 21 | B1 | C3 | farblos |
| 22 | B2 | C4 | farblos |

### BEISPIELE 23-25 FÜR KITS MIT DEN ANTISEPTISCHEN LÖSUNGEN BEISPIEL 23

Es wurde ein Kit aus den nachfolgenden Komponenten zusammengestellt. Ein Kit enthielt eine Kunststoffflasche A, die eine Septum im Flaschenkopf enthielt, eine Kunststoffflasche C, die ebenfalls ein Septum enthielt und zwei Kunststoffhohldorne als Konnektoren und Ausspritzdüsen, welche die Septa der beiden Kunststoffflaschen durch Einstechen durchstechen können. Eine handelsübliche 500 ml Kunststoffflasche A enthielt 500 ml dest. Wasser, in denen 0,45 g Natriumchlorid, 0,143 g Calciumhypochlorit und 0,143 g Citronensäure sichtklar gelöst waren.

Die handelsübliche 500 ml Kunststoffflasche B enthielt 500 ml dest. Wasser, in denen 0,45 g Natriumchlorid, 0,700 g Natriumascorbat und 0,062 g Ascorbinsäure gelöst waren. Die Kunststoffflaschen C sowie die Kunststoffhohldorne wurden durch Gammasterilisation mit einer Dosis größer 25 kGy sterilisiert. Die Lösung in der Kunststoffflasche A wurde sterilfiltriert und in eine zuvor dampfsterilisierte Kunststoffflasche gefüllt.

### BEISPIEL 24

Ein Kit bestand aus einer Kunststoffflasche A, die eine Septum im Flaschenkopf enthielt, einer Kunststoffflasche C, die ebenfalls ein Septum enthielt und zwei Kunststoffhohldorne als Konnektoren und Ausspritzdüsen, welche die Septa der beiden Kunststoffflaschen durch Einstechen durchstechen können. Die handelsübliche 500 ml Kunststoffflasche A enthielt 500 ml dest. Wasser, in denen 0,45 g Natriumchlorid, 0,286 g Calciumhypochlorit und 0,286 g Citronensäure sichtklar gelöst waren. Die handelsübliche 500 ml Kunststoffflasche B enthielt 500 ml dest. Wasser, in denen 0,45 g Natriumchlorid, 1,400g Natriumascorbat und 0,122 g Ascorbinsäure gelöst waren. Die Kunststoffflaschen C sowie die Kunststoffhohldorne wurden durch Gammasterilisation mit einer Dosis größer 25 kGy sterilisiert. Die Lösung in der Kunststoffflasche A wurde sterilfiltriert und in eine zuvor dampfsterilisierte Kunststoffflasche gefüllt.

### BEISPIEL 25

Das Kit besteht aus einer Kunststoffflasche A, die eine Septum im Flaschenkopf enthielt, einer Kunststoffflasche C, die ebenfalls ein Septum enthielt und zwei Kunststoffhohldorne als Konnektoren und Ausspritzdüsen, welche die Septa der beiden Kunststoffflaschen durch Einstechen durchstechen können. Die handelsübliche 500 ml Kunststoffflasche A enthielt 500 ml dest. Wasser, in denen 0,45 g Natriumchlorid, 0,572 g Calciumhypochlorit und 0,572 g Citronensäure sichtklar gelöst waren. Die handelsübliche 500 ml Kunststoffflasche B enthielt 500 ml dest. Wasser, in denen 0,45 g Natriumchlorid, 2,800g Natriumascorbat und 0,248 g Ascorbinsäure gelöst waren. Die Kunststoffflaschen C sowie die Kunststoffhohldorne wurden durch Gammasterilisation mit einer Dosis größer 25 kGy sterilisiert. Die Lösung in der Kunststoffflasche A wurde sterilfiltriert und in eine zuvor dampfsterilisierte Kunststoffflasche gefüllt.

### BEISPIELE 26-28 FÜR KITS MIT DEN ANTISEPTISCHEN LÖSUNGEN

In den folgenden Beispielen wurden in 20 ml Luer-Lock-Kunststoffspritzen A und in 20 ml Luer-Lock-Kunststoffspritzen B folgende Gemische eingewogen:

| | **Befüllung der Kunststoffspritzen A** | | **Befüllung der Kunststoffspritzen B** | |
|---|---|---|---|---|
| **Beispiel** | **Ca(OCl)₂ [g]** | **Citronensäure [g]** | **Natriumascorbat [g]** | **Citronensäure [g]** |
| 26 | 0,143 | 0,143 | 0,700 | 0,062 |
| 27 | 0,286 | 0,286 | 1,400 | 0,122 |
| 28 | 0,572 | 0,572 | 2,800 | 0,248 |

Die Kunststoffspritzen enthielten in ihrem Innenraum eine Porexfilterscheibe, die mit einem Klemmring unmittelbar vor dem Spritzenkopf fixiert war. Die Calciumhypochlorit-Citronensäuregemische befanden sich in einem Hohlraum zwischen der Porexfilterscheibe und dem eingesetzten Kunststoffkolben. Die befüllten Luer-Lock-Kunststoffspritzen wurden mit einem in Figur 1 gezeigten Adapter durch Verschrauben verbunden. Der Adapter war als Kappe ausgebildet durch die ein Hohldorn geführt war. Der Hohldorn besaß an seiner Unterseite eine Anschrägung zum Durchstechen von Septa. Die Oberseite des Hohldorns, die sich auf der Oberseite des Adapters befand, besaß ein Luer-Lock-Gewinde, das zum Anschließen einer Kunststoffspritze und zum Anschließen einer Ausspritzdüse geeignet war.

Es wurde ein erstes Kit gebildet aus zwei handelsüblichen Kunststoffflaschen, die mit 500 ml einer 0,9 %igen wässrigen Natriumchlorid-Lösung befüllt waren, einer Kunststoffspritze A des Beispiels 26 mit dem einem verbundenen Adapter, einer Kunststoffspritze B des Beispiels 26 und zwei mit den Adaptern verschraubbaren Ausspritzdüsen. Alle Komponenten des Kits wurden mit einer Dosis von 25 kGy gammasterilsiert.

Es wurde ein zweites Kit gebildet aus zwei handelsüblichen Kunststoffflaschen, die mit 500 ml einer 0,9 %igen wässrigen Natriumchlorid-Lösung befüllt waren, einer Kunststoffspritze A des Beispiels 27 mit dem einem verbundenen Adapter, einer Kunststoffspritze B des Beispiels 27 und zwei mit den Adaptern verschraubbaren Ausspritzdüsen. Alle Komponenten des Kits wurden mit einer Dosis von 25 kGy gammasterilsiert.

Es wurden ein dritter Kit gebildet aus zwei handelsüblichen Kunststoffflaschen, die mit 500 ml einer 0,9 %igen wässrigen Natriumchlorid-Lösung befüllt waren, einer Kunststoffspritze A des Beispiels 28 mit dem einem verbundenen Adapter, einer Kunststoffspritze B des Beispiels 28 und zwei mit den Adaptern verschraubbaren Ausspritzdüsen. Alle Komponenten des Kits wurden mit einer Dosis von 25 kGy gammasterilisiert.

Eine Verwendung des Kits der Beispiele 26-28 erfolgte in der Weise, dass zuerst der Adapter mit der verbundenen Kunststoffspritze A durch das Septum der ersten Kunststoffflasche gestochen wurde. Danach wurde die Kunststoffflasche schräg gekippt und mit dem Spritzenkolben wurde die wässrige Natriumchlorid-Lösung angesaugt. Das in der Kunststoffflasche enthaltene Gemisch begann sich zu lösen. Die gebildete Lösung wurde zurück in die Kunststoffflasche durch Betätigung des Kolbens gespritzt. Der Vorgang wurde zweimal wiederholt, bis alle Gemischpartikel im Innenraum der Kunststoffspritze gelöst waren. Danach wurde die Kunststoffspritze abgeschraubt und eine Ausspritzdüse auf den Adapter aufgeschraubt.

Dann wurde der Adapter mit der verbundenen Kunststoffspritze C durch das Septum der zweiten Kunststoffflasche, die 500 ml wässrige Kochsalzlösung enthielt, gestochen. Danach wurde die Kunststoffflasche schräg gekippt und mit dem Spritzenkolben wurde die wässrige Natriumchlorid-Lösung angesaugt. Das in der Kunststoffflasche enthaltene Gemisch begann sich zu lösen. Die gebildete Lösung wurde zurück in die Kunststoffflasche durch Betätigung des Kolbens gespritzt. Der Vorgang wurde zweimal wiederholt, bis alle Gemischpartikel im Innenraum der Kunststoffspritze gelöst waren. Danach wurde die Kunststoffspritze abgeschraubt und eine Ausspritzdüse auf den Adapter aufgeschraubt.

Die Lösung A der ersten Kunststoffflasche wurde durch Zusammendrücken der Kunststoffflasche ausgepresst, wobei der Flüssigkeitsstrahl durch die Ausspritzdüse geführt wurde. Die zu desinfizierende Oberfläche wurde mit der gebildeten Lösung desinfiziert. Nach 2 Minuten wurde dann auf die zuvor behandelte Oberfläche ein gleiches Volumen der Lösung C gespritzt. Nach einer Einwirkungszeit von ungefähr zwei Minuten wurden die auf der Oberfläche verbliebene Flüssigkeit abgesaugt.

### BEZUGSZEICHENLISTE

- 100: Vorrichtung
- 101: Anschlusselement
- 102: Dorn
- 103: Reservoir
- 104: Wirkstoff
- 105: Ausgang des Reservoirs
- 106: Filter
- 107: Fluidikelement
- 108: Kolben
- 109: erstes Verbindungselement (zur Verbindung der Bauteile)
- 110: Düse
- 111: erste Durchführung (zur Aufnahme aus Behälter)
- 112: zweite Durchführung (zum Anschluss an Pumpe o.ä.)
- 113: zweites Verbindungselement (für Düse)
- 114: Verschluss
- 115: Hohlraum des Dorns
- 116: Halterung für Filter
- 117: Schild
- 118: Gelenk
- 150: erstes Bauteil
- 160: zweites Bauteil
- 200: Behälter
- 201: Septum
- 202: Flüssigkeit

## Patentansprüche

1. Kit zur Verwendung bei der lokalen antiseptischen Behandlung eines Patienten, wobei das Kit ein antiseptisches Oxidationsmittel und ein Reduktionsmittel umfasst, wobei das antiseptische Oxidationsmittel durch Reaktion mit dem Reduktionsmittel zersetzbar ist.

2. Kit zur Verwendung nach Anspruch 1, wobei die Verwendung ein Inkontaktbringen des Oxidationsmittels mit einer Gewebeoberfläche des Patienten und ein nachfolgendes Inkontaktbringen des Reduktionsmittels mit der Gewebeoberfläche des Patienten umfasst.

3. Kit zur Verwendung nach Anspruch 2, wobei das Inkontaktbringen des Oxidationsmittels und/oder des Reduktionsmittels mit der Gewebeoberfläche des Patienten jeweils für eine Zeitdauer von 10 Sekunden bis 300 Sekunden erfolgt.

4. Kit zur Verwendung nach einem der vorangehenden Ansprüche, wobei das Kit eine Stoffmenge des Reduktionsmittels umfasst, welche mindestens der Stoffmenge des Oxidationsmittels entspricht.

5. Kit zur Verwendung nach einem der vorangehenden Ansprüche, wobei das Oxidationsmittel ausgewählt ist aus der Gruppe bestehend aus Natriumhypochlorit, Calciumhypochlorit und Natriumchlorit.

6. Kit zur Verwendung nach einem der vorangehenden Ansprüche, wobei das Reduktionsmittel ausgewählt ist aus der Gruppe bestehend aus Ascorbinsäure, Natriumascorbat, Kaliumascorbat, Calciumascorbat, L-Cystin und Cysteamin.

7. Kit zur Verwendung nach einem der vorangehenden Ansprüche, wobei das Kit weiterhin eine Puffersubstanz umfasst, wobei die Puffersubstanz bevorzugt ausgewählt ist aus der Gruppe bestehend aus Citronensäure, Natriumdihydrogencitrat, Dinatriumhydrogencitrat, Natriumdihydrogenphosphat, Calciumdihydrogenphosphat, Natriumhydrogensulfat, Dinatriumhydrogenphosphat und Trinatriumphosphat, besonders bevorzugt Citronensäure.

8. Kit zur Verwendung nach einem der vorangehenden Ansprüche, wobei das Kit eine erste Lösung mit Hypochlorit-Ionen, Chloritionen und/oder hypochloriger Säure in einer Konzentration von insgesamt 50 ppm bis 5000 ppm umfasst.

9. Kit zur Verwendung nach einem der vorangehenden Ansprüche, wobei das Kit eine zweite Lösung mit Ascorbinsäure und/oder Ascorbat-Ionen in einer Konzentration von 50 ppm bis 10.000 ppm umfasst.

10. Kit zur Verwendung nach einem der vorangehenden Ansprüche, wobei das Kit eine erste Lösung mit dem Oxidationsmittel und/oder eine zweite Lösung mit einem Reduktionsmittel mit einer Osmolarität von jeweils mindestens 310 mOsmol/L aufweist/aufweisen.

11. Kit zur Verwendung nach einem der vorangehenden Ansprüche, wobei das Kit eine erste Lösung mit einem Oxidationsmittel und einem pH-Wert im Bereich von pH 4 bis pH 8 aufweist.

12. Kit zur Verwendung nach einem der vorangehenden Ansprüche, wobei das Kit eine zweite Lösung mit einem Reduktionsmittel und einem pH-Wert im Bereich von pH 4 bis pH 7 aufweist.

13. Kit zur Verwendung nach einem der vorangehenden Ansprüche, wobei eine erste Lösung mit dem Oxidationsmittel und/oder eine zweite Lösung mit dem Reduktionsmittel jeweils unmittelbar vor dem Inkontaktbringen mit der Gewebeoberfläche durch Lösen des Oxidationsmittels bzw. Reduktionsmittels in fester Form, gegebenenfalls gemeinsam mit einer Puffersubstanz, in Wasser hergestellt wird/werden.

14. Kit zur Verwendung nach einem der vorangehenden Ansprüche, wobei die Verwendung die Behandlung eines Krankheitszustands umfasst, der ausgewählt ist aus der Gruppe bestehend aus freiliegende Oberflächen von künstlichen Gelenken und gelenknahen Knochen- und Weichgeweben, bevorzugt im Rahmen von DAIR-Prozeduren, Wunden mit freiliegendem Knochengewebe, Knorpelgewebe, Bändern und Sehnen, intra- und postoperative Operationswunden, chronischen Wunden, Dekubiti, diabetische Ulcera, Ulcus cruris, Bisswunden, Schnittwunden, Stichwunden, Schnittwunden, Risswunden, Schürfwunden, Schussverletzungen, Splitterverletzungen, Fisteln, Abszesse und Verbrennungen ersten und zweiten Grades.

15. Kit zur Verwendung nach einem der vorangehenden Ansprüche, weiterhin umfassend ein Tensid, wobei die Verwendung das Inkontaktbringen des Tensids gemeinsam mit dem Oxidationsmittel mit einer Gewebeoberfläche des Patienten umfasst, wobei das Tensid bevorzugt ein Alkylsulfat ist.
